# FASCICULE DE BREVET EUROPEEN

(11) **EP 3 649 629 B1**
(45) Date de publication et mention de la délivrance du brevet: **07.06.2023**
(21) Numéro de dépôt: 18739953.0
(22) Date de dépôt: 02.07.2018
(51) Int. Cl.: G08B 21/04, A61B 5/00

(54) **SYSTEME, DISPOSITIF ET PROCEDE POUR SURVEILLER DE MANIERE PERSONNALISEE L'ETAT DE SANTE D'AU MOINS UN PATIENT A RISQUE**
SYSTEM, VORRICHTUNG UND VERFAHREN ZUR PERSONALISIERTEN ÜBERWACHUNG DES GESUNDHEITSZUSTANDS VON MINDESTENS EINEM GEFÄHRDETEN PATIENTEN
SYSTEM, DEVICE AND METHOD FOR THE PERSONALISED MONITORING OF THE HEALTH STATUS OF AT LEAST ONE AT-RISK PATIENT

(30) Priorité: 05.07.2017 FR 1770716
(43) Date de publication de la demande: 13.05.2020
(73) Titulaire: EVOLUCARE TECHNOLOGIES, 80800 Villers Bretonneux (FR)
(72) Inventeur: LE GUILCHER, Alexandre, 78360 Montesson (FR); GILBERT, Jerome, 92300 Levallois Perret (FR)
(74) Mandataire: Plasseraud IP
(86) Numéro de dépôt international: PCT/IB2018/054908
(87) Numéro de publication internationale: WO 2019/008502

(56) Documents cités:
- EP-A2- 2 688 005
- US-A1- 2014 259 414
- US-A1- 2015 106 121

## Description

### Domaine technique

L'invention se situe dans le domaine des dispositifs et des systèmes d'information médicaux.

### Etat de la technique antérieure

Des études internationales font état de taux de mortalité anormalement élevés dans la plupart des pays après des interventions chirurgicales ne devant pas être particulièrement à risque. D'autres études établissent une corrélation entre le nombre de décès post-opératoires par établissement et le nombre d'infirmières affectées à la surveillance des patients à leur retour en chambre. Encore d'autres études montrent que le suivi régulier de l'état physiologique, voire psychologique, des patients ayant été atteint d'une maladie grave limitait la probabilité de rechute et l'impact de la rechute le cas échéant par une prise en charge plus précoce. Par ailleurs il existe une pression économique pour réduire le personnel infirmier affecté à la surveillance des patients opérés et au suivi des personnes ayant quitté les établissements de soins, pour augmenter la part de chirurgie ambulatoire dans les actes chirurgicaux et de manière générale pour réduire les temps de séjour dans les hôpitaux. L'ensemble de ces raisons appellent des solutions techniques pour automatiser au moins partiellement la surveillance des patients. Parmi les solutions connues, celles qui sont basées sur l'utilisation d'équipements de monitorage hospitalier sont trop coûteuses et trop invasives, celles qui sont basées sur l'utilisation d'objets connectés grand public ne sont pas assez fiables et celles qui utilisent des dispositifs médicaux connectés standards ne permettent pas d'obtenir la spécificité et la sensibilité demandées par les professionnels de la santé qui souhaitent à la fois éviter les fausses alertes tout en n'occasionnant aucune perte de chance pour leurs patients. Des exemples de modes de réalisation de l'état de l'art peuvent être trouvés dans les documents US2014/259414 et US2015/106121A1.

### Exposé de l'invention

Le but de la présente invention est de remédier au moins partiellement aux problèmes évoqués précédemment en proposant des solutions techniques utilisables dans des établissements de soins et/ou à domicile pour surveiller l'état de santé de personnes à risque dans le but de générer automatiquement et à bon escient une alerte appropriée.

Selon un premier aspect, l'invention concerne un système pour surveiller de manière personnalisée l'état de santé d'au moins un patient à risque tel que défini dans la revendication 1 annexée, dans le but de générer automatiquement et à bon escient des alertes appropriées destinées à des professionnels de la santé et/ou à des aidants. Le système comprend :
- au moins un moyen pour fournir des informations de surveillance à un système d'information, l'au moins un moyen étant pilotable par ledit système d'information en cours de surveillance en fonction de la situation clinique d'un patient et/ou de son évolution, et/ou d'au moins une réponse à au moins une question précédente. L'au moins un moyen étant en outre associée à un patient déterminé au moyen d'un identifiant unique. L'au moins un moyen est pilotable en fonction de la situation clinique initiale d'un patient et/ou de son évolution, c'est à dire pilotable au titre de la configuration initiale du dispositif pour que son fonctionnement à venir soit adapté au contexte médical du cas clinique du patient mais aussi, le cas échéant, que le dispositif soit pilotable à tout moment en cours de surveillance d'un patient donné. Par exemple pour réaliser des mesures de vérification d'un ou de plusieurs paramètres ou de manifestations physiologiques d'une défaillance d'au moins une fonction vitale du patient surveillé en fonction de résultats de mesures précédentes ou pour poser des questions plus pertinentes par prise en compte de réponses à des questions précédentes selon des règles prédéterminées mises en oeuvre par logiciel dans le système de surveillance selon l'invention;
- au moins une donnée de personnalisation spécifique à chaque patient ayant été préalablement au moins en partie prédéterminée et/ou validée par une intelligence médicale. Ladite donnée étant associée à un patient déterminé au moyen d'un identifiant unique, l'au moins une donnée de personnalisation étant issue d'un préréglage du système de surveillance par un professionnel de la santé et/ou de l'importation dans le système de surveillance selon l'invention de données externes concernant le patient à surveiller, ou au moins un autre patient présentant des similarités avec le patient à surveiller. Ceci pour améliorer l'adaptation du fonctionnement de la surveillance au contexte unique de chaque patient pour in fine augmenter la sensibilité et/ou la spécificité de la détection d'une anomalie pouvant mettre en péril les fonctions vitales du patient et nécessitant une intervention urgente d'un professionnel de la santé. On entend par intelligence médicale, une intelligence humaine compétente sur le plan médical et/ou une intelligence artificielle ayant été approuvée par une autorité pour une utilisation médicale;
- un système d'information exécutant un programme de surveillance personnalisée pour chaque patient à partir de données issues de l'au moins un moyen pour fournir des informations de surveillance, et en fonction de l'au moins une donnée de personnalisation, ledit système d'information générant une alerte si au moins une condition est considérée par ledit système comme anormale pour le patient concerné. La notion de système d'information selon l'invention recouvre toutes les architectures techniquement pertinentes par exemple une architecture centralisée où les traitements sont exécutés en totalité dans un ordinateur ou dans des serveurs, ou une architecture plus ou moins répartie comprenant des composants programmatiques exécutés dans des terminaux et/ou dans tout ou partie desdits moyens pour fournir des informations de surveillance à un système d'information;
- au moins un moyen pour alerter au moins un professionnel de la santé et/ou au moins un aidant lorsqu'une alerte est générée par ledit système d'information ou par l'au moins un moyen pour fournir des informations de surveillance. Selon les variantes de mise en oeuvre, il s'agit de moyens d'alertes fixes comme par exemple une tablette ou un ordinateur standard dédié à la mise en oeuvre du système selon l'invention, ou une application spécifique exécutée par un équipement informatique standard partagé avec d'autres usages, ou encore un équipement spécifique dédié à la mise en oeuvre de l'invention. Un moyen d'alerte fixe étant avantageusement situé dans un lieu où la présence permanente d'un professionnel de la santé est organisée, par exemple dans le poste de surveillance infirmier d'un service hospitalier. Il est aussi prévu une mise en oeuvre de l'invention utilisant des moyens techniques de type interphone solidaires du bâtiment. Les variantes de mise en oeuvre préférées de l'invention utilisent des terminaux mobiles pour alerter les professionnels de la santé et/ou les aidants. L'aidant est en général la première personne à alerter dans le cas d'une surveillance au domicile du patient, par exemple après une intervention en chirurgie ambulatoire. Selon les variantes de mise en oeuvre ou selon le paramétrage du système selon l'invention, le système prévient un ou plusieurs aidants par des moyens d'alerte locaux ou par le biais d'un numéro de téléphone préalablement enregistré. L'aidant prévenu ayant la charge le cas échéant de prévenir un professionnel de la santé ou un service d'intervention médicale en urgence. Dans d'autres variantes de mise en oeuvre de l'invention, le système alerte l'au moins un aidant pour qu'il prenne en charge l'accompagnement humain mais aussi le système alerte en parallèle un professionnel de la santé qui prend les dispositions médicales nécessaires compte tenu des informations transmises par le système de surveillance sur l'état du patient.

Le système selon l'invention se distingue en outre en ce que l'au moins une donnée de personnalisation spécifique à chaque patient est au moins une donnée en rapport avec un réglage et/ou une validation effectué par un professionnel de la santé ou par une intelligence artificielle approuvée par une autorité pour une utilisation médicale, et/ou est au moins une donnée en rapport avec au moins une pathologie affectant le patient, et/ou est au moins une donnée en rapport avec au moins une information contenue dans un système d'information hospitalier, et/ou est au moins une donnée en rapport avec au moins une prise de médicament par le patient, et/ou est au moins une donnée en rapport avec un examen biologique du patient, et/ou est au moins une donnée en rapport avec un examen radiologique du patient, et/ou est au moins une donnée en rapport avec une caractéristique physique du patient, et/ou est au moins une donnée en rapport avec une caractéristique génétique ou un antécédent familial du patient.

Ces données étant obtenues à des niveaux biologiques multiples allant du séquençage des gènes à l'expression des protéines et des structures métaboliques, ces données peuvent couvrir tous les mécanismes impliqués dans les variations qui se produisent dans les réseaux cellulaires et qui influencent le fonctionnement des systèmes organiques dans leur totalité.

Selon un exemple non compris dans le cadre des revendications annexées, ledit système d'information comprend en outre au moins une boucle de rétroaction exploitant au moins une qualification d'alerte validée par un professionnel de la santé ou par une intelligence artificielle approuvée par une autorité pour une utilisation médicale, pour améliorer automatiquement la sensibilité et/ou la spécificité de la surveillance. L'invention prévoit l'utilisation de la rétroaction dans le cadre de l'amélioration de la surveillance du même patient. L'invention prévoit aussi l'utilisation de la rétroaction dans le cadre de l'amélioration de la surveillance d'autres patients que celui dont l'alerte a fait l'objet d'une validation médicale. Ceci par exemple en extrayant des données issues de la surveillance et/ou du paramétrage de la surveillance du patient dont l'alerte a été validée par un médecin, des données caractéristiques d'une situation clinique donnée ou d'un contexte patient prédéterminé qui sont utilisables dans le cadre de la surveillance d'autres patients ayant une situation clinique similaire ou un contexte au moins en partie partagé.

Selon un autre exemple, ledit système d'information comprend au moins un sous-ensemble logiciel exploitant des données relatives au patient à surveiller qui ont été acquises préalablement à la surveillance automatisée de son état de santé. Il s'agit d'améliorer la sensibilité et/ou la spécificité de la détection d'une anomalie pour un patient donné en exploitant des données préalablement acquises sur ce patient pour étalonner les algorithmes de détection d'anomalie et/ou pour fixer des seuils de déclenchement d'alerte au-dessus de valeurs devant être considérées comme normales pour ce patient.

Selon un autre exemple, ledit système d'information comprend en outre au moins un algorithme prédictif de génération d'alerte s'appuyant sur l'exploitation de signaux faibles et/ou de données issues de la surveillance antérieure de patients présentant au moins une caractéristique similaire. De manière générale, plus la détection d'un problème de santé est précoce, plus sa prise en charge est efficace. L'invention prévoit ainsi d'exploiter des signaux avant-coureurs qui ne sont pas encore suffisamment significatifs en tant que tels pour générer une alerte mais que des analyses préalables portant sur un très grand nombre de cas de patients ont permis d'associer à une forte probabilité de survenue postérieure d'un problème justifiant une alerte anticipée.

Selon un autre exemple, ledit système d'information comprend en outre un sous-ensemble logiciel de gestion de patients visant à faciliter la personnalisation initiale de la surveillance d'un patient par l'importation d'au moins un paramètre de réglage initial du système commun à des patients partageant au moins une caractéristique commune. Il s'agit par exemple d'un sous-ensemble logiciel de gestion de profils de patients, chaque profil étant par exemple associé à une pathologie ou a une combinaison fréquente de pathologies de sorte que la simple sélection du nom de la pathologie ou de la combinaison fréquente de pathologies dans le dossier d'un patient prédétermine instantanément au moins un réglage approprié du système de surveillance, ou une combinaison de réglages et d'options fonctionnelles appropriées le cas échéant. Des réglages plus fins pouvant être le cas échéant requis à partir d'un préréglage automatique de base. Ces raffinements de l'invention sont avantageux en ce qu'ils font gagner du temps aux professionnels de la santé en charge du paramétrage du système de surveillance pour un nouveau patient et réduisent significativement les risques d'erreur.

Le système selon l'invention se distingue en ce que ledit système d'information comprend en outre au moins un traitement informatique associant des informations de surveillance d'un patient, au moins une donnée de personnalisation spécifique à ce patient, et au moins une règle spécifique à au moins une pathologie.

Le système selon l'invention se distingue en ce que ledit système d'information comprend en outre des moyens pour exporter et des moyens pour importer des données représentatives de connaissances acquises par le système au cours de son fonctionnement, lesdites données étant associées au paramétrage ou au réglage du système et/ou à des profils cliniques et/ou à au moins une règle spécifique à au moins une pathologie et/ou à des comportements prédictifs du système. Ce raffinement de l'invention vise à permettre la transmission d'un savoir acquis par le système de surveillance au cours de son fonctionnement pour par exemple en faire bénéficier d'autres systèmes du même type, neufs ou récemment mis en fonction. Ces fonctionnalités du système mises en oeuvre par logiciel sont aussi utilisables pour partager et/ou pour uniformiser l'expérience acquise par des systèmes de surveillance au sein d'un même groupement hospitalier ou encore pour valoriser les connaissances acquises par le système de surveillance d'un exploitant.

Selon un autre exemple, ledit système d'information comprend en outre au moins deux agents logiciels de surveillance à raison d'un agent logiciel par patient surveillé. Lesdits agents logiciels étant des composants programmatiques indépendants exécutés de manière autonome dans leurs environnements contextuels respectifs de sorte que chaque patient bénéficie d'une entité logicielle de surveillance autonome qui lui est entièrement dédiée.

Selon un autre exemple, lesdits agents logiciels de surveillance coopèrent entre eux pour que toute amélioration de la sensibilité et/ou de la spécificité de la détection d'une condition d'alerte obtenue au niveau d'un agent logiciel dans des conditions données, bénéficie à au moins un autre agent logiciel exécutant sa surveillance dans des conditions au moins en partie similaires. L'invention prévoit de réaliser des transferts d'information entre agents logiciels pour tous types de similarités au moins partielles. Par exemple des similarités portant sur une pathologie ou sur une combinaison de pathologies, sur un ou plusieurs paramètres physiologiques ou sur la manifestation physiologique d'une défaillance d'une fonction vitale du patient surveillé, sur la prise d'un médicament ayant un principe actif identique ou produisant les mêmes effets, sur un terrain, des antécédents ou des prédispositions génétiques du patient, sur le contexte clinique etc.

Selon un autre exemple, ledit système d'information comprend en outre au moins un sous-ensemble logiciel d'intelligence artificielle pour améliorer la spécificité et/ou une sensibilité de la détection d'au moins une condition d'alerte. On entend par intelligence artificielle dans le contexte de l'invention, tout sous-ensemble logiciel imitant ou remplaçant l'expert médical humain dans certaines mises en oeuvre de ses fonctions cognitives.

Selon un autre exemple, ledit système d'information comprend en outre au moins deux sous-ensembles logiciels d'intelligence artificielle, présentant au moins une diversité, dont les sorties sont combinées pour améliorer la spécificité et/ou la sensibilité de la génération d'alerte. En effet, compte tenu de limitations, de spécialisations fonctionnelles, de différences d'efficacité face à un problème donné des algorithmes utilisés dans les sous-ensembles logiciels d'intelligence artificielle, l'invention prévoit de traiter les problèmes complexes et multifactoriels de la surveillance des patients en combinant de manière appropriée les résultats issus de sous-ensembles logiciels d'intelligence artificielle qui présentent au moins une différence entre eux pour obtenir en combinaison de meilleurs résultats que ce que peuvent offrir chacun des sous-ensembles logiciels d'intelligence artificielle pris séparément.

Selon un autre exemple, ladite diversité de l'au moins deux intelligences artificielles porte sur les sources de données les alimentant, et/ou sur les conditions initiales préalables à leur exécution, et/ou sur leur principe de fonctionnement, et/ou sur leur association à des patients distincts mais présentant des similitudes et/ou des différences prédéterminées.

Selon un autre exemple, ledit système d'information complète l'alerte générée par une information caractérisant le degré d'urgence de l'intervention requise en réponse à ladite alerte et/ou renseignant le professionnel de la santé sur des valeurs et/ou des tendances de tout ou partie des informations impliquées dans la génération de ladite alerte. Ce raffinement de l'invention permet aux professionnels de la santé recevant l'alerte d'arbitrer entre plusieurs actions en fonction de leurs priorités relatives et de commencer à réfléchir aux soins les plus appropriés à prodiguer au patient pendant le déplacement jusqu'au patient.

Selon un autre exemple, ledit système d'information complète l'alerte générée par la fourniture d'au moins une consigne à suivre qui est en rapport avec la cause de l'alerte. Ce type de consigne connu des professionnels de la santé sous l'appellation « conduite à tenir » vise à permettre une intervention rapide de professionnels de la santé les plus proches du patient à l'instant de la génération de l'alerte, ces professionnels n'ayant pas nécessairement toute l'expertise médicale requise mais pouvant réaliser des premiers gestes de mise en sécurité du patient avant l'arrivée d'autres professionnels disposant d'un niveau d'expertise plus élevé relativement au cas clinique du patient concerné par l'alerte. L'invention prévoit que la nature et/ou le degré de détail des consignes données à la personne qui reçoit l'alerte soit aussi en rapport avec les caractéristiques techniques du terminal utilisé. Selon les capacités de restitution du terminal, sont prévus par exemple une indication textuelle succincte affichée sur un écran, un message audio, une séquence vidéo etc. Il est aussi prévu dans des variantes de mise en oeuvre plus élaborées de proposer un tutoriel en rapport avec l'alerte permettant à du personnel de santé peu qualifié de délivrer les premiers soins ou de préparer le patient sans le mettre en danger avant l'arrivée d'un professionnel de la santé plus qualifié. Ce raffinement de l'invention est également approprié lorsque la personne alertée est un aidant qui n'a pas nécessairement les connaissances pour mettre le patient en sécurité avant l'arrivée d'un professionnel de la santé.

Selon un autre exemple, l'au moins un moyen pour alerter au moins un professionnel de la santé et/ou au moins un aidant détermine l'au moins un professionnel de la santé et/ou l'au moins un aidant à alerter, en fonction de la distance physique qui le sépare du patient objet de l'alerte et/ou de la durée du trajet pour atteindre ledit patient et/ou de la disponibilité dudit professionnel de la santé et/ou de l'aidant à l'instant de l'alerte et/ou d'un critère de compétence métier dudit professionnel de la santé. Ce raffinement de l'invention vise à optimiser le ciblage des alertes vers le ou les professionnels les plus à même d'y répondre en utilisant des données annexes telles que par exemple la localisation de chaque professionnel potentiel pouvant être alerté, la localisation du patient concerné par l'alerte, des informations de qualification rattachées à l'identifiant du professionnel de la santé dans une base de données.

Selon un autre exemple, l'au moins un moyen pour alerter au moins un professionnel de la santé comprend au moins un sous-ensemble logiciel pour traiter au moins un acquittement du professionnel de la santé et/ou de l'aidant ayant pris en charge une alerte. Il s'agit par exemple d'un acquittement de prise en charge de l'alerte dont la réception par le système permet notamment d'interrompre des alarmes sonores et/ou un mécanisme d'escalade dans la recherche d'un professionnel de la santé susceptible d'intervenir. Il s'agit aussi dans certaines variantes de mise en oeuvre d'un second type d'acquittement permettant au professionnel de la santé ayant répondu à l'alerte de signaler au système que la cause de l'alerte a été traitée. Dans des variantes de mise en oeuvre particulièrement sophistiquées de l'invention, le système relance l'alerte si le ou les acquittements attendus dans des temps maximums prédéterminés n'ont pas été reçus.

Selon un autre exemple,le système comprend au moins un sous-ensemble logiciel pour assurer la traçabilité des alertes et/ou de leur prise en charge. Il s'agit par exemple de mémoriser continûment des données représentant l'horodatage de la prise en charge et/ou le cas échéant de l'acquittement de l'alerte et un identifiant du professionnel de la santé ayant pris en charge ladite alerte et/ou du nom d'un aidant déclaré intervenant potentiel pour un patient donné.

Selon un autre exemple, l'au moins un moyen pour alerter au moins un professionnel de la santé et/ou au moins un aidant utilise un terminal standard dont des caractéristiques techniques le rende aussi utilisable pour d'autres usages. On entend par « standard » des appareils électroniques dont les caractéristiques techniques n'ont pas été déterminées pour fonctionner exclusivement dans le cadre du système selon l'invention mais pour être conforme à des standards techniques de l'industrie par exemple des terminaux de type pager, des téléphones conformes au standard DECT connectés à un réseau privé, des téléphones cellulaires ou des smartphones connecté au réseau public standard sans fil, par exemple le smartphone personnel ou professionnel du professionnel de la santé ou de l'aidant. Il est prévu que le système selon l'invention communique directement avec ledit terminal standard, ou par l'intermédiaire d'une passerelle ou d'un sous-système appropriés placés entre ledit système d'information selon l'invention et lesdits terminaux standards.

Selon un autre exemple, l'au moins un moyen pour alerter au moins un professionnel de la santé et/ou au moins un aidant utilise un terminal dont au moins une caractéristique technique est spécifique audit système. Il s'agit par exemple d'un logiciel spécifique au système selon l'invention exécuté par un ordinateur, une tablette numérique ou un smartphone standard du marché. Selon les variantes de mise en oeuvre, il s'agit aussi d'un appareil électronique spécifique au système selon l'invention. Il est prévu que le système selon l'invention communique directement avec ledit terminal spécifique, ou par l'intermédiaire d'une passerelle ou d'un sous-système appropriés placés entre ledit système d'information et lesdits terminaux spécifiques.

Le système selon l'invention se distingue en outre en ce que l'au moins un moyen pour fournir des informations de surveillance à un système d'information comprend au moins un moyen pour recevoir les réponses du patient surveillé à des questions qui lui sont posées par l'intermédiaire d'un terminal, le pilotage par logiciel dudit moyen pour fournir des informations de surveillance en cours de surveillance étant l'adaptation de tout ou partie des questions posées au patient en fonction de sa situation clinique et/ou de son évolution, et/ou d'au moins une réponse à au moins une question précédente. L'invention prévoit plusieurs variantes de mise en oeuvre pour offrir l'ergonomie la mieux adaptée aux principales catégories de patients utilisateurs. Par exemple une application exécutable sur smartphones ou sur tablettes standards, par exemple sous les systèmes d'exploitation mobiles Android (marque déposée de Google Inc.) ou iOS (marque déposée d'Apple Inc.), est prévue en tant que support des interactions système-patient pour les catégories de patients habituées à l'utilisation de ces appareils. La richesse fonctionnelle de ces appareils permet d'envisager de nombreuses manière de poser les questions et de recevoir les réponses du patient, jusqu'à des formes ludiques utilisant des animations visuelles pour les patients les plus jeunes. Pour les catégories de patients moins à leur aise avec l'informatique nomade, une version web exécutable sur microordinateurs standard PC ou MAC est prévue. Pour les catégories de patients réfractaires aux nouvelles technologies, une interface vocale utilisable avec tous les types de téléphones est prévue. Un logiciel de synthèse vocale énonce les questions et un logiciel de reconnaissance vocale multi-locuteurs reconnait les réponses simples à base de mots clés qui sont attendues. Une version simplifiée est aussi prévue pour recevoir les réponses aux questions par appui sur les touches du clavier du téléphone qui sont proposées dans les questions.

Le système selon l'invention se distingue en ce que l'au moins un moyen pour fournir des informations de surveillance audit système d'information comprend en outre au moins un objet connecté audit système. Ledit objet connecté permettant au système de recevoir des informations sans risque qu'elles soient altérées par le patient. Il s'agit d'informations quantitatives associées au patient surveillé comme son poids, sa pression artérielle, sa température etc. ou des informations qualitatives comme un indicateur d'activité physique. Ce raffinement offre les avantages d'avoir à poser moins de questions au patient et d'éviter des erreurs de lecture ou de saisie, ou encore les biais psychologiques dans les réponses. La connexion entre l'objet connecté et le système selon l'invention est le plus souvent indirecte, car les objets communicants courants utilisent une connexion sans fil à basse puissance et courte portée, par exemple conforme aux standards Bluetooth ou Wi-Fi. Dans ces cas, la communication transite par un smartphone ou une tablette, par une box ADSL ou à fibre optique ou par un point d'accès public ou privé d'un réseau d'infrastructure.

Le système selon l'invention se distingue en outre en ce que l'au moins un moyen pour fournir des informations de surveillance à un système d'information comprend un dispositif communicant dont au moins une partie est placée à proximité ou au contact d'au moins un patient pour surveiller au moins un paramètre physiologique et/ou au moins une manifestation physiologique d'une défaillance d'au moins une fonction vitale du patient surveillé, ledit dispositif communicant étant pilotable à distance par ledit système d'information pour adapter son fonctionnement à l'état clinique de l'au moins un patient surveillé et/ou à son évolution.

Le système selon l'invention se distingue en outre en ce que l'au moins un paramètre physiologique et/ou l'au moins une manifestation physiologique d'une défaillance d'au moins une fonction vitale du patient surveillé est un ensemble de de deux à six paramètres physiologiques et/ou de manifestations physiologiques élémentaires prédéterminées et/ou de paramètres environnementaux et/ou d'informations contextuelles, surveillés en combinaison, permettent de détecter une altération des fonctions vitales de la personne surveillée, le cas échéant après des traitements informatiques locaux et/ou distants appropriés de tout ou partie des données associées auxdits paramètres et/ou manifestations physiologiques.

Le système selon l'invention se distingue en outre en ce que tout ou partie desdits paramètres ou manifestations physiologiques élémentaires sont extraits de mesures physiques issues de capteurs dont le nombre est inférieur à celui desdits paramètres ou desdites manifestations physiologiques. Il est en effet prévu dans l'invention d'exploiter de manière avantageuse des capteurs, grâce notamment à des logiciels de traitement du signal appropriés pour permettre à un même capteur ou à un même sous-ensemble d'acquisition de fournir plusieurs paramètres ou manifestations physiologiques élémentaires. Par exemple un ensemble d'électrodes et son électronique d'interface permet de fournir au moins le rythme cardiaque et le rythme respiratoire du patient, un capteur optique utilisant deux longueurs d'onde permet de fournir le pouls et la saturation pulsée en oxygène.

Un autre exemple concerne un dispositif pour surveiller de manière personnalisée l'état de santé d'au moins un patient à risque de défaillance des fonctions vitales, ou à risque de décompensation, ou à risque de complications, ou à risque de récidive ou de rechute d'une pathologie. L'invention prévoit que ledit dispositif soit sédentaire c'est à dire qu'il reste attaché au lieu où est soigné ou où réside l'au moins un patient, ou mobile c'est à dire pouvant être au moins en partie porté par le patient lorsqu'il se déplace au sein du lieu de convalescence ou de résidence ou même dans le cas de certaines variantes de mise en oeuvre, à l'extérieur de tout lieu prédéterminé. Dans le cas des variantes mobiles du dispositif, il est avantageusement prévu de gérer en outre des informations de géolocalisation pour localiser le patient.

Le dispositif selon l'invention se distingue en ce qu'il comprend :
- au moins un capteur associé à la mesure d'au moins un paramètre physiologique et/ou à la détection d'au moins une manifestation physiologique d'une défaillance d'au moins une fonction vitale du patient surveillé;
- au moins un sous-ensemble d'émission de données pour transmettre des données de surveillance et/ou d'alerte à un système d'information distant, et/ou directement à des terminaux pour alerter au moins un professionnel de la santé et/ou au moins un aidant, et/ou à un sous-système intermédiaire pilotant des terminaux;
- au moins un sous-ensemble de réception de données pour recevoir d'un système d'information distant, au moins une donnée de personnalisation et/ou au moins une télécommande et/ou les données d'au moins un composant programmatique exécutable par le dispositif;
- au moins un sous-ensemble de traitement numérique comprenant au moins un microprocesseur, au moins une mémoire dans laquelle est stockée au moins un programme, au moins une mémoire vive permettant l'exécution de l'au moins un programme, et des interfaces appropriées. Il s'agit par exemple d'une interface avec l'au moins un capteur, et/ou avec l'au moins un sous-ensemble d'émission de données, et/ou avec l'au moins un sous-ensemble de réception de données et/ou avec tout autre bloc fonctionnel embarqué dont par exemple au moins un bouton, au moins un voyant lumineux, un buzzer, une batterie pour surveiller l'autonomie de l'appareil etc.;
- au moins une mémoire pour stocker au moins un identifiant unique associé à un patient et/ou tout ou partie de l'au moins une donnée de personnalisation et/ou les données de l'au moins un composant programmatique exécutable par le dispositif. Cette mémoire amovible ou non amovible étant comprise dans l'au moins un sous-ensemble de traitement numérique. Selon les variantes de mise en oeuvre, l'au moins une mémoire au sens de l'invention est un espace de stockage dédié à des données de personnalisation dans une ou plusieurs mémoires partagées avec d'autres utilisations au sein de l'au moins un sous-ensemble de traitement numérique ou il s'agit d'au moins une mémoire dédiée à cette utilisation;
- au moins un sous-ensemble d'alimentation en lien avec une source d'énergie interne et/ou externe. La source d'énergie qui selon les variantes de mise en oeuvre est embarquée lorsque la mobilité ou l'absence de fils est recherchée ou externe lorsque le dispositif de surveillance peut être branché sans inconvénient sur le réseau électrique, ou sur un réseau de communication de données permettant en outre l'alimentation énergétique des terminaux connectés, ou encore par exemple sur la source d'alimentation autonome d'un lit médicalisé. Des variantes sont aussi prévues comprenant à la fois une connexion à une source d'énergie externe et une source d'énergie embarquée de secours en cas d'interruption de la fourniture d'énergie externe.

Selon un autre exemple, l'au moins un capteur, l'au moins un paramètre physiologique ou l'au moins une manifestation physiologique d'une défaillance d'au moins une fonction vitale du patient surveillé, l'au moins un sous-ensemble d'émission de données, l'au moins un sous-ensemble de réception de données, l'au moins une donnée de personnalisation, l'au moins une télécommande, l'au moins un composant programmatique exécutable par le dispositif, l'au moins un sous-ensemble de traitement numérique, l'au moins une mémoire et l'au moins un sous-ensemble d'alimentation en lien avec une source d'énergie, sont respectivement :
- un capteur de température en contact avec la peau, un capteur de température sans contact avec la peau utilisant le rayonnement infrarouge corporel émis, un capteur de signaux électriques corporels émis par le biais d'au moins deux électrodes placées en contact avec la peau, un capteur d'impédance corporelle par le biais d'au moins deux électrodes placées en contact avec la peau, un capteur de déformation mécanique par le biais d'un matériau élastique dont une caractéristique électrique varie en fonction de l'élongation, un capteur acoustique maintenu en contact avec le corps, un capteur acoustique placé à distance du corps, un capteur optique d'absorption relative d'au moins deux longueur d'onde lumineuses, un capteur optique de réflexion relative d'au moins deux longueur d'onde lumineuses, un capteur de pression associé à la compression d'une artère, un capteur de vélocimétrie doppler, un capteur de mouvement rendu solidaire du corps ou d'un membre du patient comme par exemple un accéléromètre à un ou plusieurs axes et/ou un gyroscope pour surveiller l'activité physique du patient, un appareil électronique communicant tel qu'un smartphone incluant de tels capteurs et pouvant être utilisé par le dispositif en tant que sous-ensemble capteur déporté, une caméra avec un logiciel d'analyse d'image approprié, un capteur d'ondes cérébrales, des capteurs de paramètres environnementaux, des capteurs d'informations contextuelles;
- la température corporelle, le rythme cardiaque, le rythme respiratoire, des signes mécaniques d'une défaillance d'au moins une fonction vitale, par exemple une détresse respiratoire, tels qu'un balancement thoraco-abdominal ou un tirage des tissus mous sous le niveau des os les supportant, une sollicitation de muscles respiratoires accessoires, la saturation veineuse en oxygène, la pression artérielle, le débit cardiaque, au moins un signe clinique de la décompensation tel qu'une pâleur anormale de la peau, une cyanose des extrémités, au moins un signe clinique de complications, une transpiration anormale, des convulsions ou des mouvements anormaux, des signes cliniques anormaux du système nerveux central, une réaction anormale à une stimulation mécanique, électrique ou lumineuse, une émission vocale telle qu'au moins un cri ou un râle, une réponse verbale à une sollicitation, l'évaluation de la douleur;
- un émetteur radiofréquence unidirectionnel, ou un transmetteur radiofréquence bidirectionnel, un modem à courants porteurs en ligne utilisé en émetteur unidirectionnel ou un transmetteur à courants porteurs en ligne bidirectionnel, une interface pour connecter un câble ou une fibre optique, une interface pour transmettre des données en utilisant des rayons lumineux à une ou plusieurs longueurs d'onde visibles ou invisibles pour l'œil humain, une interface et son électronique de pilotage pour réaliser un couplage électrique, capacitif, inductif, magnétique, électromagnétique ou acoustique, en champ proche ou lointain, ledit couplage étant bidirectionnel ou unidirectionnel dans le sens dispositif vers infrastructure;
- un récepteur radiofréquence unidirectionnel, ou un transmetteur radiofréquence bidirectionnel, un modem à courants porteurs en ligne utilisé en récepteur unidirectionnel ou un transmetteur à courants porteurs en ligne bidirectionnel, une interface pour connecter un câble ou une fibre optique, une interface pour extraire des données de rayons lumineux à une ou plusieurs longueurs d'onde visibles ou invisibles pour l'œil humain, par exemple un récepteur de signaux infrarouges ou de lumière visible modulée par exemple selon le standard dit « Li-Fi »; une interface et son électronique de pilotage pour réaliser un couplage électrique, capacitif, inductif, magnétique, électromagnétique ou acoustique, en champ proche ou lointain, ledit couplage étant bidirectionnel ou unidirectionnel dans le sens infrastructure vers dispositif. Le couplage est par exemple électrique, capacitif, inductif, par des signaux sonores tels que des infrasons, du son audible ou des ultrasons, par des ondes électromagnétiques par exemple par des moyens de type NFC ou en utilisant des moyens de recharge d'un accumulateur interne par induction par exemple en démodulant la fréquence de fonctionnement de l'inducteur pour en extraire des données;
- au moins une adresse dans un réseau de communication de données, au moins un identifiant du dispositif, au moins un identifiant d'un patient associé audit dispositif, au moins une donnée en rapport avec un réglage du fonctionnement dudit dispositif pour un patient associé audit dispositif;
- une demande de mesure ou de lecture d'un état d'un capteur ou d'un sous-ensemble fonctionnel du dispositif. Par exemple la tension d'une batterie ou d'une pile alimentant tout ou partie dudit dispositif ou encore l'index d'un compteur de durée de fonctionnement pour évaluer l'autonomie restante du dispositif, une demande de mesure ou de lecture récurrente d'un état d'un capteur ou d'un sous-ensemble fonctionnel du dispositif, la transmission des données correspondante par le dispositif se faisant à une fréquence ou dans des conditions prédéterminée;
- un script, un applet, une application, un logiciel embarqué. Par exemple, dans le cas d'une architecture au moins en partie décentralisée, pour exécuter tout ou partie des traitements de surveillance, notamment des règles de génération d'une alerte à partir d'au moins une valeur de mesure et/ou une valeur d'état d'au moins un capteur dudit dispositif. Il est aussi prévu dans l'invention de pouvoir mettre à jour à distance tout ou partie du logiciel embarqué dans ledit dispositif;
- un microcontrôleur, un composant électronique dit « système sur une puce », ou un sous-ensemble modulaire standard. Par exemple une carte de traitement standard compatible PC par exemple conforme au standard PC/104, une carte de type Raspberry Pi (marque déposée de Raspberry Pi Foundation), une carte de type Arduino (marque déposée de Arduino srl), ou toute autre carte, module ou composant standard faisant fonction de sous-ensemble de traitement numérique disposant de la puissance de calcul et des interfaces nécessaires à la mise en oeuvre du dispositif selon l'invention;
- une mémoire volatile ou non volatile comprise dans un composant électronique dit « système sur une puce », dans un composant ou dans un sous-ensemble fonctionnel dédié au stockage de données, ou dans un support amovible tel qu'une carte à puce, une carte à mémoire SD, une clé USB ou tout équivalent fonctionnel ou successeur de ces supports amovibles standards de mémoire;
- un sous-ensemble électronique d'alimentation associé à au moins un accumulateur rechargeable, à au moins une pile, à au moins un supercondensateur, un récepteur d'énergie transmise sans fil en champ proche ou lointain, une connexion Ethernet de type « PoE », un sous-ensemble d'alimentation raccordé à un réseau électrique à courant alternatif ou continu ou à au moins une source d'alimentation externe. Par exemple la batterie d'un lit médicalisé autonome ou au moins un capteur d'énergie, par exemple des cellules photovoltaïques avantageusement associées à un éclairage artificiel apte à transmettre aussi des données par exemple en utilisant des techniques de communication connues sous l'appellation « VLC » ou « LiFi ».

Selon un autre exemple, le dispositif comprend en outre une commande d'appel d'urgence utilisable par l'au moins un patient surveillé. Il s'agit par exemple d'une commande manuelle avec ou sans fil de type « poire » ou pendentif équipée d'un bouton poussoir, d'un bouton activable par le patient sur l'au moins une partie du dispositif qu'il porte, d'une commande utilisant la reconnaissance de geste, d'image ou la reconnaissance vocale d'appel au secours et/ou de tout autre dispositif de commande adapté à un handicap particulier.

Selon un autre exemple, le dispositif comprend un sous-ensemble de base et au moins un sous-ensemble d'acquisition comprenant l'au moins un capteur associé à la mesure d'au moins un paramètre physiologique et/ou à la détection d'au moins une manifestation physiologique d'une défaillance d'au moins une fonction vitale du patient surveillé, ledit sous-ensemble de base et l'au moins un sous-ensemble d'acquisition communiquant par des moyens filaires ou sans fil. Par exemple ledit sous-ensemble de base et l'au moins un sous-ensemble d'acquisition communiquent en simultané ou à l'alternat au moyen d'une liaison radiofréquence ou filaire bidirectionnelle. Des variantes de mise en oeuvre économiques de l'invention sont prévues où par exemple le sous-ensemble de base transmet à l'au moins un sous-ensemble d'acquisition des informations de personnalisation avant l'entrée dans l'étape de surveillance, par exemple en utilisant des moyens tels qu'un couplage inductif utilisé en outre pour recharger un accumulateur compris dans ledit sous ensemble d'acquisition et ce dernier transmet audit sous-ensemble de base, les données collectées pendant l'étape de surveillance au moyen d'une liaison radiofréquence ou infrarouge unidirectionnelle.

Selon un autre exemple, ledit sous-ensemble de base comprend des moyens pour recharger une source d'énergie rechargeable comprise dans l'au moins un sous-ensemble d'acquisition. Dans le cas d'un sous-ensemble de base agencé pour piloter plusieurs sous-ensemble d'acquisition, ladite base est aussi agencée pour pouvoir recharger les sous-ensembles d'acquisition comprenant une batterie, ceci simultanément ou en séquence selon les variantes de mise en oeuvre.

Selon un autre exemple, ledit sous-ensemble de base comprend des moyens pour piloter tout ou partie des fonctions de l'au moins sous-ensemble d'acquisition.

Selon un autre exemple, ledit sous-ensemble de base comprend des moyens pour gérer simultanément la surveillance d'une pluralité de patients se trouvant dans un même lieu ou dans des lieux voisins à portée des moyens de communication mis en oeuvre entre ledit sous-ensemble de base et les sous-ensembles d'acquisition concernés. On entend par lieux voisins, dans la même chambre ou dans des chambres voisines séparées par au moins une paroi, ou dans des box individuels distincts compris dans une même salle de soins, ou à des emplacements individuels dans une salle de réveil, ou dans structure temporaire de type hôpital de campagne ou cellule de crise, ou dans un véhicule de type ambulance.

Selon un autre exemple, ledit sous-ensemble de base comprend des moyens pour alerter au moins un professionnel de la santé et/ou au moins un aidant lorsqu'une alerte est générée. L'invention prévoit de manière avantageuse que le sous-ensemble de base selon l'invention comprenne aussi des moyens tels qu'une alarme sonore ou un émetteur radiofréquence apte à communiquer directement avec des terminaux tels que des pagers portés par des professionnels de la santé dans un établissement de soins. Il est aussi prévu des variantes où le sous-ensemble de base comprend des moyens de connexion avec une infrastructure de télécommunication locale permettant l'utilisation de terminaux standards tels que des téléphones sans fil DECT ou des moyens de connexion avec des infrastructures télécom distantes permettant l'utilisation de téléphones cellulaires standards.

Selon un autre exemple, ledit sous-ensemble de base et/ou le sous-ensemble d'acquisition comprend des moyens pour signaler et/ou pour acquitter une alerte ayant été générée pour l'au moins un patient dont l'au moins un sous-ensemble d'acquisition est rattaché audit sous-ensemble de base. Il s'agit par exemple d'un voyant lumineux positionné à un endroit très visible du dispositif pour signaler que le ou que l'un des patients rattachés audit sous-ensemble de base à fait l'objet d'une génération d'alerte. Ceci permet en outre de localiser plus facilement un patient en détresse dans des salles de soins comprenant de nombreux dispositifs selon l'invention. Il est aussi prévu que le sous-ensemble de base comprenne aussi un moyen comme par exemple un bouton poussoir placé à son sommet pour acquitter une alerte lorsque la personne alertée est arrivée au chevet du patient. Il est aussi prévu dans les variantes où un même sous-ensemble de base supervise plusieurs sous-ensembles d'acquisition que ces derniers comprennent un moyen de signalisation permettant au professionnel de la santé de repérer rapidement le patient ayant généré l'alerte parmi les patients surveillés.

Selon un autre exemple, toute anomalie technique susceptible d'altérer la surveillance du patient est considérée par le dispositif et plus généralement par le système selon l'invention comme étant une cause de génération d'alerte. Il s'agit par exemple d'une panne matérielle ou logicielle ou encore d'une insuffisance d'alimentation électrique de tout ou partie du dispositif, d'une rupture de liaison susceptible d'empêcher la transmission de données susceptibles de générer une alerte etc. L'invention prévoit des raffinements tels que la distinction des causes d'alertes techniques et des causes d'alertes médicales de sorte que ne soient alertées respectivement que des personnes affectées à la maintenance technique du système de surveillance ou des professionnels de la santé affectés au soin des patients.

Selon un autre exemple, l'au moins un sous-ensemble d'acquisition comprend des moyens pour transmettre des informations relatives à son autonomie énergétique et/ou des moyens pour adapter tout ou partie de son fonctionnement en fonction de la quantité d'énergie restant dans la source d'énergie autonome qu'il comprend. L'autonomie de l'alimentation électrique du sous-ensemble d'acquisition peut s'avérer insuffisante et cela constitue potentiellement un risque de non génération d'alerte. L'invention prévoit, selon les variantes de mise en oeuvre, de surveiller continûment l'autonomie énergétique des sous-ensembles concernés du dispositif, voire de prolonger leur autonomie en adoptant des stratégies visant à réduire la consommation électrique tout en assurant une qualité de service suffisante pour ne pas mettre le patient en danger. Par exemple en espaçant, voire en arrêtant, les mesures les plus consommatrices d'énergie comme la mesure de saturation pulsée en oxygène dans le cas de l'utilisation de moyens optoélectroniques pour la mettre en oeuvre.

Selon un autre aspect, l'invention concerne un procédé pour permettre à un système de surveillance personnalisée de l'état de santé d'au moins un patient à risque, tel que défini dans la revendication 7 annexée.

Le procédé selon l'invention comprend les étapes :
- de réglage initial d'au moins un paramètre de fonctionnement du moyen pour fournir des informations de surveillance à un système d'information et/ou des conditions d'exécution du programme de surveillance personnalisée en fonction d'au moins une information spécifique au patient surveillé;
- de mesure récurrente d'au moins un paramètre physiologique et/ou de détection d'au moins une manifestation physiologique d'une défaillance d'au moins une fonction vitale du patient surveillé et/ou de réception d'au moins une réponse à au moins une question posée au patient surveillé et/ou de réception d'au moins une donnée transmise par un objet connecté. La nature et la fréquence desdites mesures et/ou le cas échéant l'échantillonnage des détections étant déterminées par exemple en fonction de critères liés à la pathologie du patient surveillé et/ou à son état clinique et/ou à son évolution et/ou à l'autonomie énergétique du moyen pour fournir des informations de surveillance à un système d'information le cas échéant;
- de détermination par ledit système d'information et/ou par l'au moins un moyen pour fournir des informations de surveillance, si une alerte doit être générée en fonction d'au moins un résultat d'au moins une mesure d'au moins un paramètre physiologique et/ou d'au moins une détection d'au moins une manifestation physiologique d'une défaillance d'au moins une fonction vitale du patient surveillé et/ou d'au moins une réponse à au moins une question posée au patient surveillé et/ou d'au moins une donnée transmise par un objet connecté, et en fonction d'au moins une information spécifique au patient surveillé. Cette étape qui est mise en oeuvre par logiciel est exécutée selon les variantes d'architecture en tout ou partie dans ledit système d'information et/ou dans l'au moins un moyen pour fournir des informations de surveillance à un système d'information;
- d'alerte d'au moins un professionnel de la santé et/ou d'au moins un aidant lorsqu'une alerte est générée par ledit système d'information ou par l'au moins un moyen pour fournir des informations de surveillance.

Le procédé selon l'invention se distingue en ce qu'il comprend en outre une étape d'ajustement d'au moins un paramètre de fonctionnement du moyen pour fournir des informations de surveillance à un système d'information et/ou des conditions d'exécution du programme de surveillance personnalisée en fonction de l'évolution de la situation clinique du patient surveillé. Cette étape qui est mise en oeuvre par logiciel est exécutée selon les variantes d'architecture en tout ou partie dans ledit système d'information et/ou dans l'au moins un moyen pour fournir des informations de surveillance à un système d'information.

Le procédé selon l'invention se distingue en ce qu'il comprend en outre une étape d'acquisition préalable d'au moins une information de référence. Par exemple avant un acte thérapeutique ou un évènement clinique précédant la surveillance. Cette étape du procédé selon l'invention permet d'étalonner de manière personnalisée le système selon l'invention, par exemple en acquérant tout ou partie de l'au moins un paramètre physiologique préalablement à l'acte médical qui justifie la surveillance du patient. En pratique cette étape du procédé selon l'invention est avantageusement prévue au cours de la visite médicale qui précède un acte programmée, par exemple dans le cas d'un acte chirurgical, au cours du rendez-vous préalable avec le médecin anesthésiste. Cette étape réduite à l'acquisition rapide de l'au moins un paramètre physiologique qui sera suivi par le système selon l'invention après un acte médical peut avantageusement s'inscrire aussi dans le cas d'un acte médical non programmé, par exemple dans le cadre d'une intervention en médecine d'urgence, voire en médecine de guerre où le flux de patient peut être très élevé et où un système de surveillance post-interventionnel est particulièrement adapté pour faire face à des situations de pointe où le personnel infirmier peut être rapidement en nombre insuffisant pour effectuer une surveillance efficace des patients traités.

Le procédé selon l'invention se distingue en ce qu'il comprend en outre une étape de gestion de l'acquittement et/ou du non acquittement dans un temps prédéterminé de l'alerte par l'au moins un professionnel de la santé et/ou par l'au moins un aidant alerté. Cette étape qui est mise en oeuvre par logiciel est exécutée selon les variantes d'architecture en tout ou partie dans ledit système d'information et/ou dans l'au moins un moyen pour fournir des informations de surveillance à un système d'information. L'invention prévoit une traçabilité des interventions par l'identification de l'au moins un professionnel de la santé et/ou de l'au moins un aidant qui est intervenu, ou a tenté d'intervenir, auprès du patient et par l'enregistrement de l'événement horodaté correspondant. Des raffinements sont aussi prévus pour relancer l'alerte, et le cas échéant pour gérer des mécanismes dits d'escalade vers des niveaux hiérarchiques supérieurs au sein d'une organisation si le ou les acquittements attendus dans un temps prédéterminés ne sont pas reçus.

### Brève description des dessins

D'autres avantages et caractéristiques de l'invention apparaîtront à l'examen de la description détaillée de modes de mise en oeuvre nullement limitatifs, et des dessins annexés où :
La figure 1 illustre la structure du système selon l'invention.
La figure 2 illustre une première variante du système selon l'invention.
La figure 3 illustre une seconde variante du système selon l'invention.
La figure 4 illustre une première variante d'utilisation d'intelligence artificielle.
La figure 5 illustre une combinaison d'intelligences artificielles.
La figure 6 illustre la structure du dispositif selon l'invention.
La figure 7 illustre une première variante du dispositif selon l'invention.
La figure 8 illustre une seconde variante du dispositif selon l'invention.
La figure 9 illustre le procédé selon l'invention.

### Description détaillée des figures et des modes de réalisation

D'autres particularités et avantages de l'invention apparaîtront encore dans la description ci-après ainsi que dans les dessins annexés donnés à titre d'exemples non limitatifs :

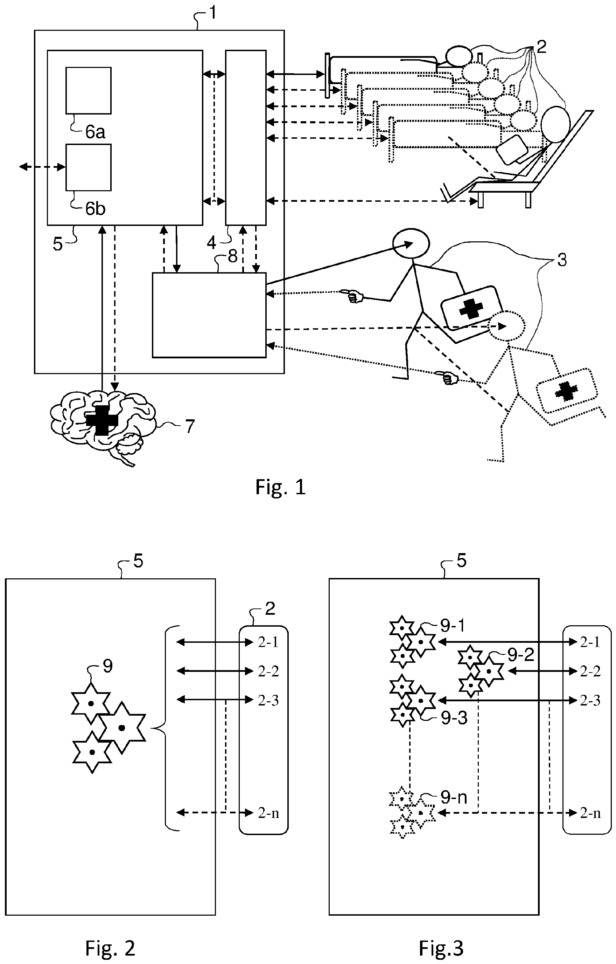

La figure **1** illustre la structure du système selon l'invention.

La structure du système selon l'invention 1 pour surveiller de manière personnalisée les fonctions vitales d'un patient 2 pour générer automatiquement et à bon escient des alertes appropriées destinées à des professionnels de la santé et/ou à des aidants 3 comprend au moins un moyen 4 pour fournir des informations de surveillance à un système d'information 5. L'au moins un moyen 4 est pilotable par ledit système d'information 5 en cours de surveillance en fonction de la situation clinique de chaque patient 2 et/ou de l'évolution de sa situation clinique, et/ou d'au moins une réponse à au moins une question précédente. L'invention prévoit que les moyens 4 soient basés sur des questions posées aux patient et complétés le cas échéant par des données fournies automatiquement au système d'information 5 par des objets connectés. Les moyens 4 sont par exemple un ou plusieurs programmes informatiques ou composants logiciels, leurs moyens d'exécution et leurs moyens de présentation aux patients pour générer des questions spécifiques à chaque patient. Les moyens de présentation des questions au patient sont par exemple une tablette numérique, un smartphone, un téléviseur connecté directement ou par un adaptateur, un téléphone, une montre, un bracelet mais aussi dans le cas d'une variante universelle de l'invention, un dispositif permettant en outre l'acquisition automatique d'informations de surveillance. Lesdits objets connectés sont par exemple une balance connectée pour suivre l'évolution du poids du patient, un tensiomètre connecté pour suivre sa pression artérielle ou encore un pilulier électronique connecté pour suivre l'observance d'un traitement prescrit, ceci sans nécessiter de saisie d'informations par le patient. La variante de l'invention qui est basée sur des questionnaires adaptatifs, et le cas échéant sur l'utilisation par le patient de petits appareils communicants facilitant le suivi de son état de santé, nécessite la possession de ses facultés cognitives. Si ce n'est pas le cas ou lorsqu'est préférée une variante dans laquelle le patient a un rôle passif, l'invention prévoit d'utiliser pour les moyens 4, un dispositif pilotable permettant l'acquisition automatique d'informations de surveillance. L'invention prévoit aussi que l'au moins un moyen 4 soit une combinaison des deux variantes principales du système comprenant à la fois au moins un dispositif pilotable de surveillance automatique de l'état de santé du patient et des questionnaires évolutifs complétés le cas échéant par des données fournies par des appareils tiers.

Le système selon l'invention comprend en outre au moins une donnée de personnalisation 6a, 6b spécifique à chaque patient. L'au moins une donnée de personnalisation ayant été préalablement au moins en partie prédéterminée et/ou validée par une intelligence médicale 7 humaine ou artificielle. L'au moins une donnée de personnalisation 6a résulte d'un réglage ou d'un paramétrage propre au système qui est réalisé ou prescrit par un professionnel de la santé ou par une intelligence artificielle médicale approuvée par les autorités en fonction des informations sur le patient à surveiller qu'il connait par exemple suite à une consultation et/ou à la lecture d'un dossier, de résultats d'analyses biologiques, de résultats d'imagerie etc. Il est aussi prévu que l'au moins une donnée de personnalisation 6a soit issue d'une étape d'étalonnage réalisée avant l'évènement clinique qui justifie la surveillance du patient, par exemple avant un acte chirurgical. Selon la variante de mise en oeuvre relativement aux moyens 4 pour fournir des informations de surveillance, l'au moins une donnée de personnalisation est au moins une réponse à au moins une question appropriée et/ou au moins une donnée de surveillance de référence issue d'au moins un moyen 4 selon l'invention. Il est aussi prévu que l'au moins une donnée de personnalisation 6b provienne d'au moins une source externe au système selon l'invention, par exemple d'un système d'information hospitalier ou d'un dossier patient externe partagé. Il est aussi prévu que soient combinées au sein du système selon l'invention, des données internes résultant d'un réglage a priori, ou d'un étalonnage impliquant le patient et d'au moins une donnée issue d'une source externe pour affiner et/ou pour sécuriser la personnalisation de la surveillance. Le système de surveillance automatique selon l'invention comprend en outre un système d'information 5 exécutant un programme de surveillance personnalisée pour chaque patient à partir de données issues de l'au moins un moyen pour fournir des informations de surveillance 4, et en fonction de l'au moins une donnée de personnalisation 6a, 6b. Ledit système d'information génère une alerte si au moins une condition est considérée par ledit système comme anormale pour le patient concerné. Le système selon l'invention comprend en outre au moins un moyen 8 pour alerter au moins un professionnel de la santé et/ou au moins un aidant 3 lorsqu'une alerte est générée par ledit système d'information 5. Il est aussi prévu des variantes de mise en oeuvre décentralisées du système selon l'invention dans lesquelles l'alerte est générée par l'au moins un moyen 4 pour fournir des informations de surveillance. Il est aussi prévu des variantes de mise en oeuvre où l'au moins un moyen 4 pour fournir des informations de surveillance comprend aussi au moins un moyen 8 pour alerter au moins un professionnel de la santé et/ou au moins un aidant 3 lorsqu'une alerte est générée.

La figure **2** illustre une première variante du système selon l'invention. Cette variante de mise en oeuvre repose sur un système d'information traditionnel dans lequel au moins un programme informatique ou au moins un composant logiciel 9 associé à une fonction déterminée gère cette fonction pour l'ensemble 2 des patients 2-1 à 2-n à surveiller.

La figure **3** illustre une seconde variante du système selon l'invention. Cette variante de mise en oeuvre repose sur un système d'information dans lequel chaque patient à surveiller 2-1 à 2-n est pris en charge par au moins un composant logiciel 9-1 à 9-n qui lui est entièrement dédié et qui comprend l'environnement d'exécution nécessaire pour gérer ledit patient de manière autonome. Il est convenu d'appeler agent logiciel de surveillance l'entité programmatique autonome 9-1 à 9-n affectée à un patient surveillé 2-1 à 2-n donné.

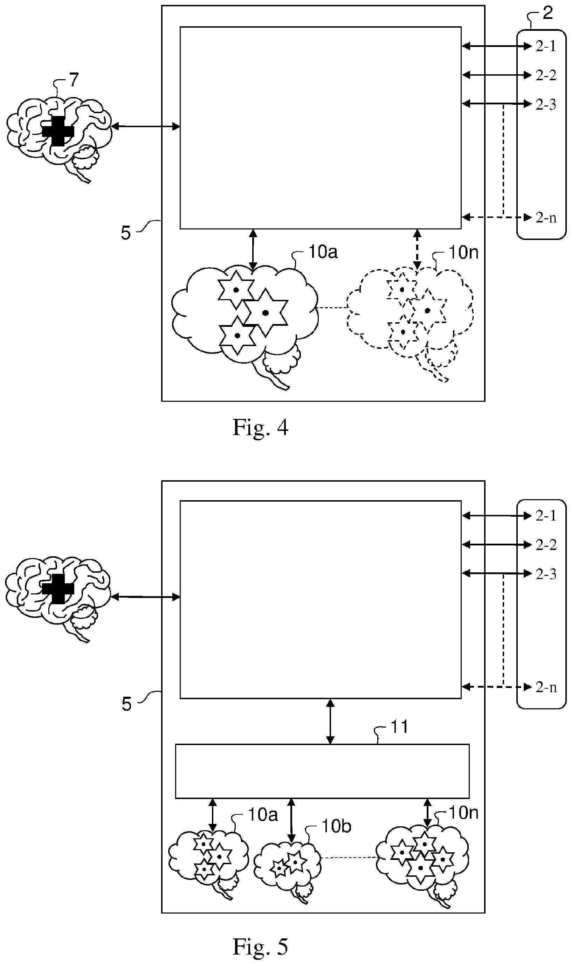

La figure **4** illustre une première variante d'utilisation d'intelligence artificielle au sein du système d'information 5. Un ou plusieurs logiciels dits d'intelligence artificielle 10a à 10n sont prévus dans le cadre dudit système d'information 5 pour améliorer les performances du système de surveillance automatisé des patients 2, en particulier sur le plan de sa sensibilité et/ou de sa spécificité. Ceci se fait en complément des algorithmes classiques de type moteur de règles qui déterminent si une alerte doit être générée en fonction de la pathologie, des informations de surveillance et des données de personnalisation telles que celles résultant d'un paramétrage initial du système pour un patient donné 2-1 à 2-n par une intelligence médicale 7. L'apport des logiciels à forte complexité logique ou algorithmique mettant en oeuvre des fonctions cognitives apparentées à celles des humains que sont les logiciels dits d'intelligence artificielle est prévu par exemple pour repérer des corrélations difficilement détectables par des moyens conventionnels dans des quantités massives de données issues d'une pluralité de patients, pour rechercher des indices ou des signes avant-coureurs d'un processus physiologique en cours risquant de conduire un patient donné à une défaillance vitale avérée, pour affiner le paramétrage et la personnalisation de la détection d'alerte en explorant automatiquement des données faiblement couplées aux processus physiologiques surveillés comme par exemple des données représentatives du terrain du patient, de ses antécédents, de prédispositions génétiques etc.

La figure **5** illustre une combinaison d'intelligences artificielles. Cet exemple de mise en oeuvre se distingue de celui de la figure 4 en ce que le système d'information 5 comprend au moins un sous-ensemble logiciel 11 qui combine les résultats d'une pluralité de logiciels d'intelligence artificielle 10a à 10n fonctionnant sur des principes différents et/ou sur des jeux de données différents pour produire des résultats combinés supérieurs sur au moins un critère qualitatif ou quantitatif, par exemple sur le plan de la réduction du risque d'erreur, sur celui de l'augmentation de la sensibilité d'une détection ou sur le plan de la puissance de calcul pour pouvoir traiter avec un temps de latence acceptable les flux de données issus des patients 2-1 à 2-n, ceci par rapport aux résultats que fourniraient les logiciels 10a à 10n exécutés seuls. Il est aussi prévu de combiner les approches qu'illustrent les figure 4 et 5 en mettant en oeuvre un ou plusieurs logiciels d'intelligence artificielle spécialisés exécutés seuls pour réaliser des tâches pour lesquelles les résultats obtenus sont jugés suffisants, et des logiciels d'intelligence artificielle complétés par un sous-ensemble logiciel de combinaison des résultats pour réaliser d'autres tâches nécessitant des performances supérieures à celles pouvant être obtenues sans combinaison.

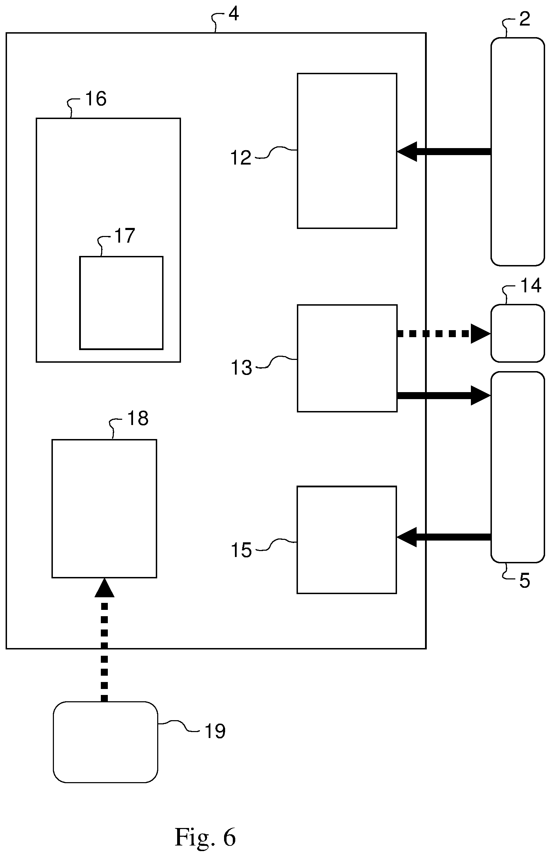

La figure **6** illustre la structure du dispositif selon l'invention. Ledit dispositif fait partie des moyens 4 selon l'invention pour fournir des informations de surveillance personnalisée de l'état de santé d'au moins un patient 2 à un système d'information 5. Le dispositif selon l'invention comprend au moins au moins un capteur 12 associé à la mesure d'au moins un paramètre physiologique et/ou à la détection d'au moins une manifestation physiologique d'une défaillance d'au moins une fonction vitale de l'au moins un patient surveillé 2. Le dispositif comprend en outre au moins un sous-ensemble d'émission de données 13 pour transmettre des données de surveillance et/ou d'alerte, à un système d'information distant 5 et/ou directement ou indirectement à des terminaux 14 pour alerter au moins un professionnel de la santé et/ou au moins un aidant. Le dispositif comprend en outre au moins un sous-ensemble de réception de données 15 pour recevoir d'un système d'information distant 5 au moins une donnée de personnalisation et/ou au moins une télécommande et/ou les données d'au moins un composant programmatique exécutable par le dispositif. Le dispositif comprend en outre au moins un sous-ensemble de traitement numérique 16, par exemple un système sur une puce à faible consommation d'énergie comprenant au moins un microprocesseur à architecture 32 ou 64 bit sous licence de la société ARM Ltd., au moins une mémoire dans laquelle est stockée au moins un programme, au moins une mémoire vive permettant l'exécution de l'au moins un programme, une interface avec l'au moins un capteur 12, une interface avec l'au moins un sous-ensemble d'émission de données 13, une interface avec l'au moins un sous-ensemble de réception de données 15, au moins une mémoire 17 pour stocker au moins un identifiant unique associé à un patient et/ou tout ou partie de l'au moins une donnée de personnalisation et/ou les données de l'au moins un composant programmatique exécutable par le dispositif. Le dispositif comprend en outre au moins un sous-ensemble d'alimentation 18 en lien avec une source d'énergie interne, par exemple une batterie Li-ion ou une pile dont la capacité est dimensionnée pour permettre au dispositif selon l'invention de surveiller un patient continûment pendant une durée de 5 à 10 jours en toute autonomie. Dans le cas d'une batterie, une connexion électrique ou un couplage inductif est par exemple prévu avec une source d'énergie externe 19 tel qu'un adaptateur basse tension connectable au réseau électrique pour recharger la batterie embarquée entre deux périodes de surveillance de patients ou la continuation de la surveillance pour un même patient.

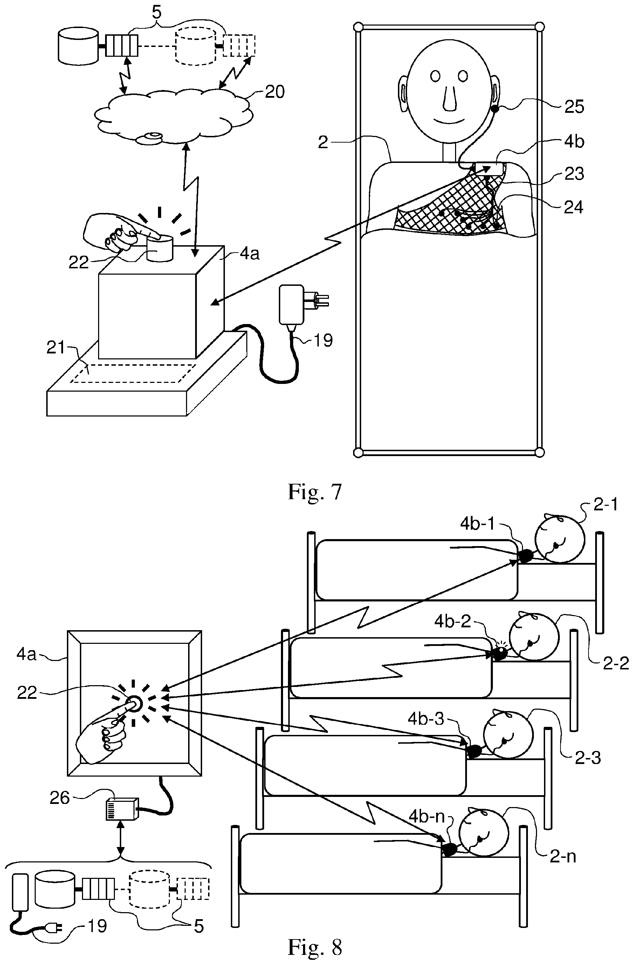

La figure **7** illustre une première variante du dispositif selon l'invention.

Il s'agit d'une première variante du dispositif selon l'invention qui repose sur la répartition des moyens et des fonctions mis en oeuvre entre un sous-ensemble de base et au moins un sous-ensemble d'acquisition comprenant l'au moins un capteur associé à la mesure d'au moins un paramètre physiologique et/ou à la détection d'au moins une manifestation physiologique d'une défaillance d'au moins une fonction vitale du patient surveillé. Cette répartition en plusieurs sous-ensembles vise principalement à optimiser le dispositif selon l'invention pour réduire l'encombrement et augmenter l'autonomie de la partie qui est au contact ou au voisinage du patient. Un autre avantage de cette répartition des ressources du dispositif est de faciliter la logistique et d'optimiser la répartition des coûts du matériel en permettant l'utilisation de sous-ensembles stériles à usage unique, le cas échéant reconditionnables, pour le matériel qui est au contact du patient. Dans ce premier exemple non limitatif, le dispositif selon l'invention est scindé en une base 4a à poser par exemple sur une table de chevet et une partie 4b qui est au contact du patient 2. Chacun des sous-ensembles comprend un sous-ensemble de traitement numérique et au moins une interface de communication appropriés. La base communique avec au moins un système d'information distant 5 par une liaison avec un réseau télécommunication 20. Selon les variantes de mise en oeuvre ou les choix de l'utilisateur, cette liaison est directe au moyen d'un modem radio embarqué compatible avec un réseau cellulaire, ou de manière indirecte par une liaison Ethernet proposée sous la forme d'un connecteur RJ45 standard ou d'une liaison Wi-Fi pouvant être établie avec un point d'accès privé tel qu'une box ADSL ou à fibre optique ou encore avec un point d'accès public. La base est en outre connectée à une source d'énergie externe 19 compte tenu du fait qu'elle réunit les sous-ensembles techniques du dispositif selon l'invention qui sont les plus consommateurs d'énergie. Une zone de recharge 21 est en outre prévue sur la base pour recharger, le cas échéant, par induction ou par connexion électrique directe le sous-ensemble d'acquisition. La base comprend en outre un bouton lumineux visible et facile d'accès. Il est prévu d'utiliser ce bouton lumineux par exemple pour signaler par un éclairage vert que le dispositif fonctionne de manière nominale, que les liaisons impliquées entre le sous-ensemble capteur et la base et entre la base et le système d'information distant sont fonctionnelles et que l'autonomie énergétique des deux sous-ensembles est suffisante. Ce bouton est en outre utilisable pour signaler localement la survenue d'une alerte par un éclairage rouge clignotant, il est aussi prévu d'accompagner ce signalement d'une alerte sonore. En cas d'alerte, une pression sur le bouton l'acquitte et signale au système que le patient est pris en charge. L'événement est horodaté et enregistré. En cours de surveillance, une pression sur le bouton est interprétée comme un appel à l'aide et génère une alerte manuelle. Tous les événements étant horodatés et enregistrés. Pour éviter toute erreur de manipulation ou un appui par inadvertance pendant le nettoyage de la chambre par exemple, un maintien du bouton appuyé pendant plusieurs secondes est nécessaire pour déclencher une alerte. Bien entendu on ne sort pas du cadre de l'invention si les fonctions de signalisation et les fonctions de commandes utilisent des moyens distincts. Dans cet exemple non limitatif, le sous-ensemble capteur est réalisé sous la forme d'un petit boitier 4b agencé pour être fixé sur une brassière 23 en textile extensible à usage unique assurant son maintien sur le corps du patient 2. Plusieurs variantes de sous-ensemble capteur et d'emplacements sont prévus, par exemple, un bracelet, une ceinture, un pendentif, un patch autocollant etc. La variante de la figure 7 est préférée car elle est bien adaptée à une utilisation prolongée en ce que le contact avec la peau n'est pas occlusif, que le choix d'un textile approprié présentant des propriétés d'élasticité et de séchage rapide permet le maintient de capteurs, la respiration de la peau et le cas échéant la toilette du patient sans enlever le dispositif. La position près de l'épaule gauche est compatible avec toute les postures, allongées, assises et debout, la gêne occasionnée au patient est minimale et au moins une partie de l'appareil reste visible dans un lit lorsque le patient est couvert par un drap. En outre cet endroit est particulièrement avantageux en ce qu'il permet d'embarquer de nombreux capteurs en minimisant, le cas échéant, la longueur des câbles par sa proximité avec les organes vitaux essentiels. Dans la variante présentée, des électrodes 24 en nombre approprié, par exemple entre 2 et 6, sont prépositionnées aux bons endroits à l'intérieur de la brassière support pour permettre le suivi du rythme cardiaque et respiratoire. La saturation pulsée en oxygène est suivie au moyen d'un capteur amovible 25 monté sur un clip de fixation sur le lobe de l'oreille qui est un endroit moins handicapant pour le patient qu'à l'extrémité d'un doigt. Des variantes encore plus sophistiquées prévoient d'embarquer un microphone de contact tourné vers la cage thoracique faisant fonction de stéthoscope électronique, un capteur de température corporelle, un bouton « SOS » d'alerte manuelle accessible au patient, un microphone orienté vers la bouche du patient et un transducteur audio orienté vers son oreille pour si nécessaire établir une liaison phonique avec un professionnel de la santé distant en cas d'alerte. La liaison bidirectionnelle entre le sous-ensemble de base et l'au moins un sous-ensemble d'acquisition utilise avantageusement une connectivité radio à faible consommation électrique ayant une portée suffisante, le standard connu sous les appellations de « Bluetooth Low Energy » et « Bluetooth Smart » est particulièrement approprié à l'utilisation selon l'invention sur les plans de la consommation électrique, de la portée, de la sécurité, des débits binaires possibles et du fait de l'existence de nombreux objets connectés utilisant ce standard qui peuvent ainsi compléter l'invention sans nécessiter de développements supplémentaires. Bien entendu d'autres standards, par exemple des variantes à faible consommation du standard Wi-Fi, ou encore des solutions propriétaires sont aussi utilisables sans sortir du cadre de l'invention. Le boitier miniature 4b, qui est avantageusement étanche pour permettre sa réutilisation après désinfection, et qui permet au patient de faire sa toilette sans risque, est fixé sur la brassière support selon les variantes par des boutons de type « pression », par des pièces en Velcro (marque déposée de la société Velcro BVBA Pic.) ou par insertion dans un logement prévu à cet effet dans le support textile. L'invention prévoit que seulement quelques modèles de brassières en textile extensible suffisent pour offrir des solutions adaptées à toutes les morphologies et à toutes les corpulences de patients homme et femme, voire d'animaux dans le cadre d'utilisations vétérinaires de l'invention qui sont aussi prévues.

La figure **8** illustre une seconde variante du dispositif selon l'invention. Cette variante se distingue de celle de la figure 7 en ce qu'elle est optimisée pour une utilisation en routine dans un établissement de soins. La base 4a est conçue pour superviser une pluralité de patients 2-1 à 2-n occupants une chambre partagée ou une salle de réveil, par exemple 2, 4 patients ou davantage. La base 4a est agencée sous la forme d'un boitier plat optimisé pour une fixation murale. La connexion à l'au moins un système d'information 5 se fait par exemple au moyen d'une connexion Ethernet conforme au standard dit PoE (Power over the Ethernet en langue anglaise). Ainsi la même connectique compacte 26 par exemple conforme au standard RJ45 ou une connectique pour raccorder directement les fils d'une telle liaison permet au sous-ensemble de base 4a, à la fois de communiquer avec l'au moins un système d'information 5 et de trouver sa source d'énergie 19 à partir de l'infrastructure de réseau local du bâtiment. Des variantes communiquant par Wi-Fi et alimentées par un adaptateur basse-tension connectable au réseau électrique secouru de l'établissement sont aussi prévues pour répondre à tous les besoins. En plus du voyant 22 équipant le sous-ensemble de base pour signaler une alerte affectant un ou plusieurs des patients surveillés rattachés à cette base, cette variante du dispositif comprend en outre sur le boitier 4b-1 à 4b-n un voyant lumineux qui émet des flashs très visibles lorsque le patient qui le porte a généré une alerte. Ceci pour que le professionnel de la santé prévenu par le système, qui se précipite dans la chambre ou dans la salle de soins concernée, localise immédiatement le patient 4b-2 qui a un problème parmi les autres patients surveillés pour le prendre en charge sans perte de temps et donc sans perte de chance pour le patient. Il est prévu de recharger les batteries comprises dans les sous-ensembles d'acquisition entre deux utilisations au moyen d'un chargeur non représenté sur la figure. S'agissant d'une variante de mise en oeuvre optimisée pour la surveillance des patients dans des établissements de soins, le chargeur est avantageusement agencé pour pouvoir recharger simultanément une pluralité de sous-ensembles d'acquisition selon l'invention, par exemple de 2 à 10 appareils en même temps. Encore plus avantageusement sur le plan de la productivité de l'établissement de soins et de la lutte contre les maladies nosocomiales, le chargeur combine la recharge simultanée par induction d'une pluralité d'appareils étanches et leur immersion dans une solution désinfectante pendant un temps approprié à la fois à la recharge complète des appareils et à leur désinfection certaine. Le chargeur est avantageusement équipé d'un détecteur de présence de liquide désinfectant en quantité suffisante interdisant la recharge, et donc la remise en service des appareils, s'ils n'ont pas été convenablement désinfectés.

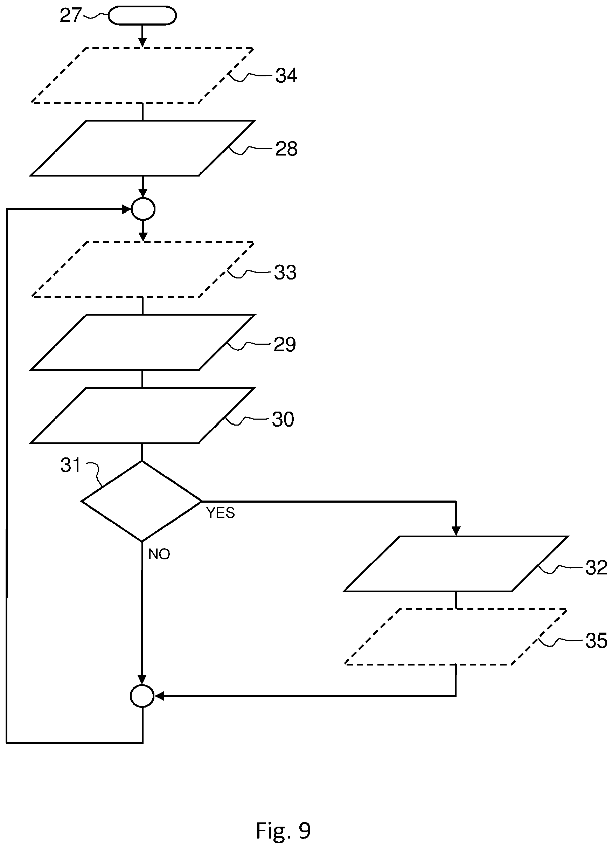

La figure **9** illustre le procédé selon l'invention. Apres l'étape 27 d'initialisation à la mise sous tension des équipements informatiques mettant en oeuvre le procédé, le procédé selon l'invention commence par l'étape 28 de réglage initial d'au moins un paramètre de fonctionnement du moyen pour fournir des informations de surveillance à un système d'information et/ou des conditions d'exécution du programme de surveillance personnalisée en fonction d'au moins une information spécifique au patient surveillé. Suit l'étape 29 de mesure récurrente d'au moins un paramètre physiologique et/ou de détection d'au moins une manifestation physiologique d'une défaillance d'au moins une fonction vitale du patient surveillé et/ou de réception d'au moins une réponse à au moins une question posée au patient surveillé et/ou de réception d'au moins une donnée transmise par un objet connecté. Vient ensuite l'étape 30 de détermination par ledit système d'information et/ou par l'au moins un moyen pour fournir des informations de surveillance, si une alerte doit être générée en fonction d'au moins un résultat d'au moins une mesure d'au moins un paramètre physiologique et/ou d'au moins une détection d'au moins une manifestation physiologique d'une défaillance d'au moins une fonction vitale du patient surveillé et/ou d'au moins une réponse à au moins une question posée au patient surveillé et/ou d'au moins une donnée transmise par un objet connecté, et en fonction d'au moins une information spécifique au patient surveillé. Si une alerte est générée, le test 31 conduit à l'étape 32 d'alerte d'au moins un professionnel de la santé et/ou d'au moins un aidant. En l'absence d'alerte, les étapes du procédé sont exécutées en boucle tout au long de la surveillance à partir de l'étape 29 ou de l'étape 33 le cas échéant. Le procédé selon l'invention comprend en outre dans certaines variantes de mise en oeuvre une étape 33 d'ajustement d'au moins un paramètre de fonctionnement du moyen pour fournir des informations de surveillance à un système d'information et/ou des conditions d'exécution du programme de surveillance personnalisée en fonction de l'évolution de la situation clinique du patient surveillé. Le procédé selon l'invention comprend en outre dans certaines variantes de mise en oeuvre une étape 34 d'acquisition préalable d'au moins une information de référence. Le procédé selon l'invention comprend en outre dans certaines variantes de mise en oeuvre une étape 35 de gestion de l'acquittement et/ou du non acquittement dans un temps prédéterminé de l'alerte par l'au moins un professionnel de la santé et/ou par l'au moins un aidant alerté.

Bien entendu, l'invention n'est pas limitée aux exemples qui viennent d'être décrits et de nombreux aménagements peuvent y être apportés sans sortir du cadre des revendications annexées,

## Revendications

1. Système (1) pour surveiller de manière personnalisée l'état de santé d'au moins un patient (2) à risque de défaillance des fonctions vitales, ou à risque de décompensation, ou à risque de complications, ou à risque de récidive ou de rechute d'une pathologie, dans le but de générer automatiquement et à bon escient des alertes appropriées destinées à des professionnels de la santé et/ou à des aidants (3), ledit système étant **caractérisé en ce qu'**il comprend :
- au moins un moyen (4) pour fournir des informations de surveillance à un système d'information (5),
∘ ledit moyen comprend un ou plusieurs récepteurs pour recevoir des réponses de patients suivis à des questions qui leur sont posées via un terminal;
∘ l'au moins un moyen (4) étant pilotable par ledit système d'information (5) en cours de surveillance en fonction de la situation clinique d'un patient et/ou de l'évolution de la situation clinique du patient, et/ou une ou plusieurs des réponses;
- un logiciel pilotant le moyen pour fournir des informations de surveillance, le pilotage par logiciel dudit moyen (4) pour fournir des informations de surveillance en cours de surveillance étant l'adaptation de tout ou partie des questions posées au patient en fonction au moins de sa situation clinique et de l'évolution de sa situation clinique, et d'au moins une réponse à au moins une question précédente, lesdites informations de surveillance
▪ concernent une réponse d'un patient suivis à des questions qui lui sont posées via le terminal et
▪ concernent un paramètre physiologique et/ou une manifestation physiologique d'une défaillance d'au moins une fonction vitale du patient surveillé,
- au moins une donnée de personnalisation (6a, 6b) spécifique à chaque patient ayant été préalablement au moins en partie prédéterminée et/ou validée par une intelligence humaine compétente sur le plan médical et/ou une intelligence artificielle ayant été approuvée par une autorité pour une utilisation médicale (7), l'au moins une donnée de personnalisation spécifique à chaque patient est au moins une donnée en rapport avec un réglage et/ou une validation effectué par un professionnel de la santé ou par une intelligence artificielle approuvée par une autorité pour une utilisation médicale, et/ou est au moins une donnée en rapport avec au moins une pathologie affectant le patient, et/ou est au moins une donnée en rapport avec au moins une information contenue dans un système d'information hospitalier, et/ou est au moins une donnée en rapport avec au moins une prise de médicament par le patient, et/ou est au moins une donnée en rapport avec un examen biologique du patient, et/ou est au moins une donnée en rapport avec un examen radiologique du patient, et/ou est au moins une donnée en rapport avec une caractéristique physique du patient, et/ou est au moins une donnée en rapport avec une caractéristique génétique ou un antécédent familial du patient;
- un système d'information (5) exécutant un programme de surveillance personnalisée pour chaque patient à partir des informations de surveillance, et en fonction de l'au moins une donnée de personnalisation, ledit système d'information générant une alerte si au moins une condition est considérée par ledit système comme anormale pour le patient concerné;
- au moins un moyen (8) pour alerter au moins un professionnel de la santé et/ou au moins un aidant (3) lorsqu'une alerte est générée par ledit système d'information (5) ou par l'au moins un moyen (4) pour fournir des informations de surveillance.

2. Système selon la revendication 1 **caractérisé en ce que** ledit système d'information comprend au moins un traitement informatique associant des informations de surveillance d'un patient, au moins une donnée de personnalisation spécifique à ce patient, et au moins une règle spécifique à au moins une pathologie.

3. Système selon l'une quelconque des revendications 1 à 2 **caractérisé en ce que** ledit système d'information comprend des moyens pour exporter et des moyens pour importer des données représentatives de connaissances acquises par le système au cours de son fonctionnement, lesdites données étant associées au paramétrage ou au réglage du système et/ou à des profils cliniques et/ou à au moins une règle spécifique à au moins une pathologie et/ou à des comportements prédictifs du système.

4. Système selon l'une quelconque des revendications 1 à 3 **caractérisé en ce que** l'au moins un moyen pour fournir des informations de surveillance à un système d'information comprend un dispositif communicant dont au moins une partie est placée à proximité ou au contact d'au moins un patient pour surveiller au moins un paramètre physiologique et/ou au moins une manifestation physiologique d'une défaillance d'au moins une fonction vitale du patient surveillé, ledit dispositif communicant étant pilotable à distance par ledit système d'information pour adapter son fonctionnement à l'état clinique de l'au moins un patient surveillé et/ou à son évolution.

5. Système selon la revendication 4 **caractérisé en ce que** l'au moins un paramètre physiologique et/ou l'au moins une manifestation physiologique d'une défaillance d'au moins une fonction vitale du patient surveillé est un ensemble de deux à six paramètres physiologiques et/ou de manifestations physiologiques élémentaires prédéterminées et/ou de paramètres environnementaux et/ou d'informations contextuelles qui, surveillés en combinaison, permettent de détecter une altération des fonctions vitales de la personne surveillée.

6. Système selon la revendication 5 **caractérisé en ce que** tout ou partie desdits paramètres ou manifestations physiologiques élémentaires sont extraits de mesures physiques issues de capteurs dont le nombre est inférieur à celui desdits paramètres ou desdites manifestations physiologiques.

7. Procédé pour permettre à un système de surveillance personnalisée de l'état de santé d'au moins un patient à risque selon l'une quelconque des revendications 1 à 5, de générer automatiquement et à bon escient une alerte destinée à des professionnels de la santé et/ou à des aidants, le procédé étant **caractérisé en ce qu'**il comprend les étapes :
- de réglage initial d'au moins un paramètre de fonctionnement du moyen pour fournir des informations de surveillance à un système d'information et/ou des conditions d'exécution du programme de surveillance personnalisée, le réglage étant adapté en tout ou partie à des questions posées au patient via un terminal en fonction au moins de sa situation clinique et de la évolution de sa situation clinique, et d'au moins une réponse à au moins une question précédente, les informations de surveillance
∘ concernent une réponse d'un patient suivis à des questions qui lui sont posées via le terminal et
∘ concernent un paramètre physiologique et/ou une manifestation physiologique d'une défaillance d'au moins une fonction vitale du patient surveillé,
- de mesure récurrente d'au moins un paramètre physiologique et/ou de détection d'au moins une manifestation physiologique d'une défaillance d'au moins une fonction vitale du patient surveillé et/ou de réception d'au moins une réponse à au moins une question posée au patient surveillé et/ou de réception d'au moins une donnée transmise par un objet connecté;
- de détermination par ledit système d'information et/ou par l'au moins un moyen pour fournir des informations de surveillance, si une alerte doit être générée en fonction d'au moins un résultat d'au moins une mesure d'au moins un paramètre physiologique et/ou d'au moins une détection d'au moins une manifestation physiologique d'une défaillance d'au moins une fonction vitale du patient surveillé et/ou d'au moins une réponse à au moins une question posée au patient surveillé et/ou d'au moins une donnée transmise par un objet connecté, et en fonction d'au moins une information spécifique au patient surveillé et en fonction d'au moins une donnée de personnalisation (6a, 6b) spécifique à chaque patient ayant été préalablement au moins en partie prédéterminée et/ou validée par une intelligence humaine compétente sur le plan médical et/ou une intelligence artificielle ayant été approuvée par une autorité pour une utilisation médicale (7), l'au moins une donnée de personnalisation spécifique à chaque patient est au moins une donnée en rapport avec un réglage et/ou une validation effectué par un professionnel de la santé ou par une intelligence artificielle approuvée par une autorité pour une utilisation médicale, et/ou est au moins une donnée en rapport avec au moins une pathologie affectant le patient, et/ou est au moins une donnée en rapport avec au moins une information contenue dans un système d'information hospitalier, et/ou est au moins une donnée en rapport avec au moins une prise de médicament par le patient, et/ou est au moins une donnée en rapport avec un examen biologique du patient, et/ou est au moins une donnée en rapport avec un examen radiologique du patient, et/ou est au moins une donnée en rapport avec une caractéristique physique du patient, et/ou est au moins une donnée en rapport avec une caractéristique génétique ou un antécédent familial du patient;
- d'alerte d'au moins un professionnel de la santé et/ou d'au moins un aidant lorsqu'une alerte est générée par ledit système d'information ou par l'au moins un moyen pour fournir des informations de surveillance.

8. Procédé selon la revendication 7 **caractérisé en ce qu'**il comprend en outre une étape d'ajustement d'au moins un paramètre de fonctionnement du moyen pour fournir des informations de surveillance à un système d'information et/ou des conditions d'exécution du programme de surveillance personnalisée en fonction de l'évolution de la situation clinique du patient surveillé.

9. Procédé selon l'une quelconque des revendications 7 ou 8 **caractérisé en ce qu'**il comprend en outre une étape d'acquisition préalable d'au moins une information de référence.

10. Procédé selon l'une quelconque des revendications 7 à 9 **caractérisé en ce qu'**il comprend en outre une étape de gestion de l'acquittement et/ou du non acquittement dans un temps prédéterminé de l'alerte par l'au moins un professionnel de la santé et/ou par l'au moins un aidant alerté.

## Patentansprüche

1. System (1) zur personalisierten Überwachung des Gesundheitszustands mindestens eines Patienten (2), bei dem die Gefahr von Versagen der Vitalfunktionen, oder die Gefahr von Dekompensation, oder die Gefahr von Komplikationen, oder die Gefahr von Wiederauftreten oder Rückfall einer Erkrankung besteht, mit dem Ziel, automatisch und aus gutem Grund angemessene Alarme zu erzeugen, die für medizinische Fachkräfte und/oder für Hilfskräfte (3) bestimmt sind, wobei das System **dadurch gekennzeichnet ist, dass** es umfasst:
- mindestens ein Mittel (4) zum Liefern von Überwachungsinformationen an ein Informationssystem (5),
- wobei das Mittel einen oder mehrere Empfänger umfasst, um Antworten von überwachten Patienten auf Fragen, die ihnen über ein Endgerät gestellt werden, zu empfangen;
- wobei das mindestens eine Mittel (4) vom Informationssystem (5) während der Überwachung in Abhängigkeit von der klinischen Situation eines Patienten und/oder der Entwicklung der klinischen Situation des Patienten, und/oder einer oder mehrerer der Antworten gesteuert werden kann,
- eine Software, die das Mittel zum Liefern von Überwachungsinformationen steuert, wobei es sich bei der Softwaresteuerung des Mittels (4) zum Liefern von Überwachungsinformationen während der Überwachung um die Anpassung aller oder eines Teils der Fragen, die dem Patienten gestellt werden, in Abhängigkeit von mindestens seiner klinischen Situation und der Entwicklung seiner klinischen Situation, und von mindestens einer Antwort auf mindestens eine vorhergehende Frage handelt, wobei die Überwachungsinformationen:
- eine Antwort eines überwachten Patienten auf Fragen betreffen, die ihm über das Endgerät gestellt werden, und
- einen physiologischen Parameter und/oder eine physiologische Manifestation eines Versagens mindestens einer Vitalfunktion des überwachten Patienten betreffen,
- mindestens eine für jeden Patienten spezifische Datenangabe (6a, 6b) zur Personalisierung, die zuvor mindestens zum Teil von einer menschlichen Intelligenz, die im medizinischen Bereich kompetent ist, und/oder einer künstlichen Intelligenz, die von einer Behörde für eine medizinische Verwendung (7) zugelassen wurde, vorbestimmt und/oder validiert wurde, wobei es sich bei der mindestens einen für jeden Patienten spezifischen Datenangabe zur Personalisierung um mindestens eine Datenangabe in Zusammenhang mit einer Einstellung und/oder einer Validierung handelt, die von einer medizinischen Fachkraft oder von einer künstlichen Intelligenz, die von einer Behörde für eine medizinische Verwendung zugelassen wurde, vorgenommen wurde, und/oder um mindestens eine Datenangabe in Zusammenhang mit mindestens einer Erkrankung handelt, an der der Patient leidet, und/oder um mindestens eine Datenangabe in Zusammenhang mit mindestens einer Information handelt, die in einem Krankenhausinformationssystem enthalten ist, und/oder um mindestens eine Datenangabe in Zusammenhang mit mindestens einer Medikamenteneinnahme durch den Patienten handelt, und/oder um mindestens eine Datenangabe in Zusammenhang mit einer biologischen Untersuchung des Patienten handelt, und/oder um mindestens eine Datenangabe in Zusammenhang mit einer radiologischen Untersuchung des Patienten handelt, und/oder um mindestens eine Datenangabe in Zusammenhang mit einem körperlichen Merkmal des Patienten handelt, und/oder um mindestens eine Datenangabe in Zusammenhang mit einem genetischen Merkmal oder einer familiären Veranlagung des Patienten handelt,
- ein Informationssystem (5), das ein für jeden Patienten auf Grundlage der Überwachungsinformationen und in Abhängigkeit von der mindestens einen Datenangabe zur Personalisierung personalisiertes Überwachungsprogramm ausführt, wobei das Informationssystem einen Alarm erzeugt, wenn mindestens eine Bedingung vom System als für den betreffenden Patienten anormal erachtet wird,
- mindestens ein Mittel (8) zum Alarmieren mindestens einer medizinischen Fachkraft und/oder mindestens einer Hilfskraft (3), wenn vom Informationssystem (5) oder von dem mindestens einen Mittel (4) zum Liefern von Überwachungsinformationen ein Alarm erzeugt wird.

2. System nach Anspruch 1, **dadurch gekennzeichnet, dass** das Informationssystem mindestens eine informatische Verarbeitung umfasst, die Überwachungsinformationen eines Patienten, mindestens eine für diesen Patienten spezifische Datenangabe zur Personalisierung, und mindestens eine für mindestens eine Erkrankung spezifische Regel miteinander verknüpft.

3. System nach einem der Ansprüche 1 bis 2, **dadurch gekennzeichnet, dass** das Informationssystem Mittel zum Exportieren und Mittel zum Importieren von Daten umfasst, die für Kenntnisse repräsentativ sind, welche vom System im Laufe seines Betriebs erfasst wurden, wobei die Daten mit der Parametrierung oder mit der Einstellung des Systems und/oder mit klinischen Profilen und/oder mit mindestens einer für mindestens eine Erkrankung spezifischen Regel und/oder mit prädiktiven Verhalten des Systems verknüpft sind.

4. System nach einem der Ansprüche 1 bis 3, **dadurch gekennzeichnet, dass** das mindestens eine Mittel zum Liefern von Überwachungsinformationen an ein Informationssystem eine kommunizierende Vorrichtung umfasst, von der mindestens ein Teil in der Nähe oder in Kontakt mit mindestens einem Patienten angebracht ist, um mindestens einen physiologischen Parameter und/oder mindestens eine physiologische Manifestation eines Versagens mindestens einer Vitalfunktion des überwachten Patienten zu überwachen, wobei die kommunizierende Vorrichtung vom Informationssystem ferngesteuert werden kann, um ihren Betrieb an den klinischen Zustand des mindestens einen überwachten Patienten und/oder an dessen Entwicklung anzupassen.

5. System nach Anspruch 4, **dadurch gekennzeichnet, dass** es sich bei dem mindestens einen physiologischen Parameter und/oder der mindestens einen physiologischen Manifestation eines Versagens mindestens einer Vitalfunktion des überwachten Patienten um einen Satz von zwei bis sechs physiologischen Parametern und/oder vorbestimmten elementaren physiologischen Manifestationen und/oder Umweltparametern und/oder Kontextinformationen handelt, die, wenn sie in Kombination überwacht werden, ermöglichen, eine Veränderung der Vitalfunktionen der überwachten Person zu erkennen.

6. System nach Anspruch 5, **dadurch gekennzeichnet, dass** alle oder ein Teil der Parameter oder elementaren physiologischen Manifestationen aus physikalischen Messungen extrahiert werden, die von Sensoren stammen, deren Anzahl kleiner ist als die der Parameter oder der physiologischen Manifestationen.

7. Verfahren, um einem System zur personalisierten Überwachung des Gesundheitszustands mindestens eines gefährdeten Patienten nach einem der Ansprüche 1 bis 5 zu ermöglichen, automatisch und aus gutem Grund einen Alarm zu erzeugen, der für medizinische Fachkräfte und/oder für Hilfskräfte bestimmt ist, wobei das Verfahren **dadurch gekennzeichnet ist, dass** es die Schritte umfasst:
- des anfänglichen Einstellens mindestens eines Betriebsparameters des Mittels zum Liefern von Überwachungsinformationen an ein Informationssystem und/oder der Ausführungsbedingungen des Programms zur personalisierten Überwachung, wobei die Einstellung ganz oder zum Teil auf Fragen, die dem Patienten über ein Endgerät gestellt werden, in Abhängigkeit von mindestens seiner klinischen Situation und der Entwicklung seiner klinischen Situation und von mindestens einer Antwort auf mindestens eine vorhergehende Frage angepasst wird, wobei die Überwachungsinformationen:
- eine Antwort eines überwachten Patienten auf Fragen betreffen, die ihm über das Endgerät gestellt werden, und
- einen physiologischen Parameter und/oder eine physiologische Manifestation eines Versagens mindestens einer Vitalfunktion des überwachten Patienten betreffen,
- des wiederkehrenden Messens mindestens eines physiologischen Parameters und/oder des Erkennens mindestens einer physiologischen Manifestation eines Versagens mindestens einer Vitalfunktion des überwachten Patienten und/oder des Empfangens mindestens einer Antwort auf mindestens eine Frage, die dem überwachten Patienten gestellt wurde, und/oder des Empfangens mindestens einer Datenangabe, die von einem verbundenen Objekt übertragen wird,
- des Bestimmens, durch das Informationssystem und/oder durch das mindestens eine Mittel zum Liefern von Überwachungsinformationen, ob ein Alarm erzeugt werden soll in Abhängigkeit von mindestens einem Ergebnis mindestens einer Messung mindestens eines physiologischen Parameters und/oder mindestens eines Erkennens mindestens einer physiologischen Manifestation eines Versagens mindestens einer Vitalfunktion des überwachten Patienten und/oder mindestens einer Antwort auf mindestens eine Frage, die dem überwachten Patienten gestellt wurde, und/oder mindestens einer Datenangabe, die von einem verbundenen Objekt übertragen wird, und in Abhängigkeit von mindestens einer für den überwachten Patienten spezifischen Information und in Abhängigkeit von mindestens einer für jeden Patienten spezifischen Datenangabe (6a, 6b) zur Personalisierung, die zuvor mindestens zum Teil von einer menschlichen Intelligenz, die im medizinischen Bereich kompetent ist, und/oder einer künstlichen Intelligenz, die von einer Behörde für eine medizinische Verwendung (7) zugelassen wurde, vorbestimmt und/oder validiert wurde, wobei es sich bei der mindestens einen für jeden Patienten spezifischen Datenangabe zur Personalisierung um mindestens eine Datenangabe in Zusammenhang mit einer Einstellung und/oder einer Validierung handelt, die von einer medizinischen Fachkraft oder von einer künstlichen Intelligenz, welche von einer Behörde für eine medizinische Verwendung zugelassen wurde, vorgenommen wurde, und/oder um mindestens eine Datenangabe in Zusammenhang mit mindestens einer Erkrankung handelt, an der der Patient leidet, und/oder um mindestens eine Datenangabe in Zusammenhang mit mindestens einer Information handelt, die in einem Krankenhausinformationssystem enthalten ist, und/oder um mindestens eine Datenangabe in Zusammenhang mit mindestens einer Medikamenteneinnahme durch den Patienten handelt, und/oder um mindestens eine Datenangabe in Zusammenhang mit einer biologischen Untersuchung des Patienten handelt, und/oder um mindestens eine Datenangabe in Zusammenhang mit einer radiologischen Untersuchung des Patienten handelt, und/oder um mindestens eine Datenangabe in Zusammenhang mit einem körperlichen Merkmal des Patienten handelt, und/oder um mindestens eine Datenangabe in Zusammenhang mit einem genetischen Merkmal oder einer familiären Veranlagung des Patienten handelt,
- des Alarmierens mindestens einer medizinischen Fachkraft und/oder mindestens einer Hilfskraft, wenn vom Informationssystem oder von dem mindestens einen Mittel zum Liefern von Überwachungsinformationen ein Alarm erzeugt wird.

8. Verfahren nach Anspruch 7, **dadurch gekennzeichnet, dass** es weiter einen Schritt des Anpassens mindestens eines Betriebsparameters des Mittels zum Liefern von Überwachungsinformationen an ein Informationssystem und/oder der Ausführungsbedingungen des Programms zur personalisierten Überwachung in Abhängigkeit von der Entwicklung der klinischen Situation des überwachten Patienten umfasst.

9. Verfahren nach einem der Ansprüche 7 oder 8, **dadurch gekennzeichnet, dass** es weiter einen Schritt des vorhergehenden Erfassens mindestens einer Referenzinformation umfasst.

10. Verfahren nach einem der Ansprüche 7 bis 9, **dadurch gekennzeichnet, dass** es weiter einen Schritt des Verwaltens der Quittierung und/oder der Nichtquittierung des Alarms innerhalb einer vorbestimmten Zeit durch die mindestens eine medizinische Fachkraft und/oder durch die mindestens eine Hilfskraft, die alarmiert wurde, umfasst.

## Claims

1. A system (1) for personalised monitoring of the health status of at least one patient (2) at risk of vital function failure, or at risk of decompensation, or at risk of complications, or at risk of recurrence or relapse of a pathology, for purposes of automatically astutely generating appropriate alerts intended for health professionals and/or for caregivers (3), said system being **characterised in that** it comprises:
- at least one means (4) for providing monitoring information to an information system (5),
∘ said means comprise one or more receivers for receiving responses of patients being followed to questions asked thereto via a terminal;
∘ the at least one means (4) being drivable by said information system (5) during the monitoring as a function of the clinical situation of a patient and/or the change over time in the clinical situation of the patient, and/or one or more of the replies;
- a software driving the means for providing monitoring information, driving by software of said means (4) for providing monitoring information during the monitoring being the adaptation of all or part of the questions asked to the patient as a function of at least his/her clinical situation and the change over time in his/her clinical situation, and of at least one reply to at least one preceding question, said monitoring information:
▪ relates to a reply of a patient being followed to questions asked thereto via the terminal and
▪ relates to a physiological parameter and/or a physiological manifestation of a failure of at least one vital function of the patient being monitored,
- at least one piece of personalisation data (6a, 6b) specific to each patient having previously being determined at least in part and/or validated by a medically skilled human intelligence and/or an artificial intelligence having been approved by an authority for a medical use (7), the at least one piece of personalisation data specific to each patient is at least one piece of data in relation to a setting and/or a validation performed by a health professional or by an artificial intelligence approved by an authority for a medical use, and/or is at least one piece of data in relation to at least one pathology affecting the patient, and/or is at least one piece of data in relation to at least one piece of information contained in a hospital information system, and/or is at least one piece of data in relation to at least one medication intake by the patient, and/or is at least one piece of data in relation to a biological examination of the patient, and/or is at least one piece of data in relation to a radiological examination of the patient, and/or is at least one piece of data in relation to a physical characteristic of the patient, and/or is at least one piece of data in relation to a genetic characteristic or a family history of the patient;
- an information system (5) running a personalised monitoring program for each patient from the monitoring information, and as a function of the at least one piece of personalisation data, said information system generating an alert if at least one condition is considered by said system as being abnormal for the patient concerned;
- at least one means (8) for alerting at least one health professional and/or at least one caregiver (3) when an alert is generated by said information system (5) or by the at least one means (4) for providing monitoring information.

2. The system according to claim 1 **characterised in that** said information system comprises at least one computing processing associating monitoring information of a patient, at least one piece of personalisation data specific to this patient, and at least one rule specific to at least one pathology.

3. The system according to any of claims 1 to 2 **characterised in that** said information system comprises means for exporting and means for importing data representative of knowledge acquired by the system during its operation, said data being associated with the parameterisation or setting of the system and/or with clinical profiles and/or with at least one rule specific to at least one pathology and/or with predictive behaviours of the system.

4. The system according to any of claims 1 to 3 **characterised in that** the at least one means for providing monitoring information to an information system comprises a communicating device at least one part of which is placed in proximity to or in contact with at least one patient to monitor at least one physiological parameter and/or at least one physiological manifestation of a failure of at least one vital function of the patient being monitored, said communicating device being remotely drivable by said information system to adapt its operation to the clinical status of the at least one patient being monitored and/or to its change over time.

5. The system according to claim 4 **characterised in that** the at least one physiological parameter and/or the at least one physiological manifestation of a failure of at least one vital function of the patient being monitored is a set of two to six predetermined elementary physiological parameters and/or physiological manifestations and/or environmental parameters and/or contextual information which, monitored in combination, make it possible to detect a deterioration in the vital functions of the person being monitored.

6. The system according to claim 5 **characterised in that** all or part of said elementary physiological parameters or manifestations are extracted from physical measurements from sensors the number of which is less than that of said physiological parameters or manifestations.

7. A method for enabling a system for personalised monitoring of the health status of at least one at-risk patient according to any of claims 1 to 5, to automatically astutely generating an alert intended for health professionals and/or for caregivers, the method being **characterised in that** it comprises the steps of:
- initially setting at least one operation parameter of the means for providing monitoring information to an information system and/or conditions for running the personalised monitoring program, the setting being adapted in whole or part to questions asked to the patient via a terminal as a function of at least his/her clinical situation and the change over time in his/her clinical situation, and to at least one reply to at least one preceding question, the monitoring information:
∘ relates to a response of a patient being followed to questions asked thereto via the terminal and
∘ relates to a physiological parameter and/or a physiological manifestation of a failure of at least one vital function of the patient being monitored,
- recurrently measuring at least one physiological parameter and/or detecting at least one physiological manifestation of a failure of at least one vital function of the patient being monitored and/or receiving at least one reply to at least one question asked to the patient being monitored and/or receiving at least one piece of data transmitted by a connected object;
- determining by said information system and/or by the at least one means for providing monitoring information, whether an alert should be generated as a function of at least one result of at least one measurement of at least one physiological parameter and/or at least one detection of at least one physiological manifestation of a failure of at least one vital function of the patient being monitored and/or at least one reply to at least one question asked to the patient being monitored and/or at least one piece of data transmitted by a connected object, and as a function of at least one piece of information specific to the patient being monitored and as a function of at least one piece of personalisation data (6a, 6b) specific to each patient having been previously predetermined at least in part and/or validated by a medically skilled human intelligence and/or an artificial intelligence having been approved by an authority for a medical use (7), said at least one piece of personalisation data specific to each patient is at least one piece of data in relation to a setting and/or a validation performed by a health professional or by an artificial intelligence approved by an authority for a medical use, and/or is at least one piece of data in relation to at least one pathology affecting the patient, and/or is at least one piece of data in relation to at least one piece of information contained in a hospital information system, and/or is at least one piece of data in relation to at least one medication intake by the patient, and/or is at least one piece of data in relation to a biological examination of the patient, and/or is at least one piece of data in relation to a radiological examination of the patient, and/or is at least one piece of data in relation to a physical characteristic of the patient, and/or is at least one piece of data in relation to a genetic characteristic or a family history of the patient;
- alerting at least one health professional and/or at least one caregiver when an alert is generated by said information system or by the at least one means for providing monitoring information.

8. The method according to claim 7 **characterised in that** it further comprises a step of adjusting at least one operation parameter of the means for providing monitoring information to an information system and/or conditions for running the personalised monitoring program as a function of the change over time in the clinical situation of the patient being monitored.

9. The method according to any of claims 7 or 8 **characterised in that** it further comprises a step of acquiring at least one piece of reference information beforehand.

10. The method according to any of claims 7 to 9 **characterised in that** it further comprises a step of managing the acknowledgment and/or non-acknowledgement of the alert within a predetermined time by the at least one health professional and/or by the at least one caregiver alerted.
